# EUROPÄISCHE PATENTSCHRIFT

(11) **EP 3 976 150 B1**
(45) Veröffentlichungstag und Bekanntmachung des Hinweises auf die Patenterteilung: **11.02.2026**
(21) Anmeldenummer: 20729624.5
(22) Anmeldetag: 18.05.2020
(51) Int. Cl.: A61M 16/00, A61M 16/10

(54) **ATEMTHERAPIEGERÄT UND SYSTEME**
RESPIRATORY THERAPY DEVICE AND SYSTEMS
APPAREIL DE THÉRAPIE RESPIRATOIRE ET SYSTÈMES

(30) Priorität: 25.05.2019 DE 102019003710
(43) Veröffentlichungstag der Anmeldung: 06.04.2022
(73) Patentinhaber: Löwenstein Medical Technology S.A., 2557 Luxembourg (LU)
(72) Erfinder: SCHATTNER, Jan, 25421 Pinneberg (DE); GÖBEL, Christof, 22457 Hamburg (DE)
(74) Vertreter: Löwenstein Medical IP
(86) Internationale Anmeldenummer: PCT/EP2020/025229
(87) Internationale Veröffentlichungsnummer: WO 2020/239252

(56) Entgegenhaltungen:
- WO-A1-2008/028247
- WO-A2-2005/035021
- US-A- 4 257 415
- US-A1- 2007 045 152
- US-A1- 2009 301 482
- US-A1- 2010 059 055
- US-A1- 2010 229 867
- US-A1- 2012 145 151
- US-A1- 2012 152 255
- US-A1- 2012 240 932
- US-A1- 2014 102 449
- US-A1- 2015 023 782
- US-A1- 2015 320 954
- US-A1- 2018 085 541
- US-A1- 2018 250 482
- US-B2- 9 707 371
- US-B2- 9 795 752

## Beschreibung

Die vorliegende Erfindung betrifft ein Atemtherapiegerät zum Erzeugen einer Atemluftströmung für die Durchführung einer Atemtherapie. Das Atemtherapiegerät umfasst wenigstens eine Gehäuseeinrichtung zur Einhausung von Gerätekomponenten. Die Erfindung betrifft zudem eine Koppeleinrichtung zur Kopplung von wenigstens zwei solchen Atemtherapiegeräten.

Für einen möglichst komfortablen Einsatz von Atemtherapiegeräten zur Beatmung oder Hustenunterstützung sind der Platzbedarf und die Zugänglichkeit bzw. Bedienbarkeit des Geräts sehr wichtig. Besonders bei der Beatmung im Bett oder im Rollstuhl steht oft nur sehr wenig Platz zum Abstellen des Geräts zur Verfügung. Zugleich muss das Gerät aber auch so aufgestellt werden, dass die Bedienelemente gut zugänglich sind und die auf einem Display dargestellten Informationen gut erkannt werden können. Hinzukommt, dass sich die Position des Anwenders ändert und sich die Stellung des Geräts ebenfalls anpassen können soll.

US 2007/045152 A1 offenbart ein Aufbewahrungssystem für ein Gerät, das einen Vorrat an unter Druck stehender Atemluft an einen Patienten abgibt, umfasst einen Behälter mit einem ersten Teil und einem zweiten Teil.

US 2012/152255 A1 offenbart Systeme, Vorrichtungen und Verwendungsmethoden, die für die Behandlung von Atemwegserkrankungen oder schlafbezogener Atmung geeignet sind, umfassen eine Patientenschnittstelle, die so beschaffen ist, dass sie bei der Verwendung an einem Teil des Gesichts eines Patienten befestigt und gegen diesen abgedichtet wird. Ein Strömungsgenerator kann mit der Patientenschnittstelle verbunden und an einem Patienten angeschlossen werden.

US 9 707 371 B2 offenbart ein Beatmungsgerät umfassend einen Kompressor, einen Vorratsbehälter, eine Ventil-Anordnung, die mit dem Kompressor, dem Vorratsbehälter und einer Inhalationsleitung in Verbindung steht. Ein Steuergerät steuert die Ventil Baugruppe zwischen einer Speicherkonfiguration, in der Beatmungsgas vom Kompressor in den Speicherbehälter geliefert wird, und einer Abgabekonfiguration, in der Beatmungsgas vom Speicherbehälter zur Inhalationsleitung geliefert wird.

US 2012/240932 A1 offenbart ein Gerät mit einem Gebläse, das so konfiguriert ist, dass es eine Zufuhr von Atemgas bereitstellt, und ein Zuführungsschlauch, der so konfiguriert ist, dass er die Zufuhr von Atemgas an ein Benutzerateminterface liefert.

US 2010/059055 A1 offenbart ein Gasabgabesystem mit einem Gehäuse und einem Strömungsgenerator, der eine Gebläsebaugruppe und eine Isolierbaugruppe zur Verbindung des Strömungsgenerators mit dem Gehäuse umfasst.

D6 US 2014/102449 A1 offenbart eine Montageeinheit zur Verwendung mit einer tragbaren Gebläsevorrichtung und ein System einer einfachen tragbaren Gebläsevorrichtung, die mit einer Vielzahl von Montagevorrichtungen austauschbar ist. Die tragbare Gebläsevorrichtung verfügt über einen Gasflussgenerator zur Bereitstellung eines Gasstroms von zu einer Maske für die Zufuhr eines Gasflusses zu einem Atemweg eines Patienten.

WO 2005/035021 A2 offenbart ein Gasverabreichungsverfahren zur Verabreichung von Gas an einen Atemweg eines Patienten mit einem Nasenvorhof und zur Verwendung mit einer Gasverabreichungsvorrichtung umfassend eine primäre Gasquelle, die zur Bereitstellung von Gas betrieben werden kann, und ein Nasenvorhofteil, das so angeordnet ist, dass es das Gas von der primären Gasquelle aufnimmt.

US 2015/320954 A1 offenbart ein CPAP-Gerät, welches die Bequemlichkeit der Handhabung verbessert und die Belastungen für einen Patienten reduziert.

US 2009/301482 A1 offenbart eine Gasabgabevorrichtung und ein System und Verfahren zur Abgabe von befeuchtetem Druckgas durch eine Leitung an einen Patienten.

US 2015/023782 A1 offenbart einen Strömungsgenerator umfassend ein Gehäuse und ein Gebläse, das so strukturiert ist, dass es einen Strom von unter Druck stehender Atemluft erzeugt und eine Aufhängevorrichtung, die das Gebläse innerhalb des Gehäuses hält und eine Druckdichtung zwischen der Niederdruck- und der Hochdruckseite des Gebläses bereitstellt.

US 2018/250482 A1 offenbart ein PAP-System zur Erzeugung einer Zufuhr von Druckluft, eine Patientenschnittstelle, einen Luftzufuhrschlauch, um die Patientenschnittstelle und die PAP-Vorrichtung miteinander zu verbinden und eine Abdeckung, die zumindest einen Teil der PAP-Vorrichtung und einen Teil des Luftzufuhrschlauchs im Wesentlichen umschließt. Die Abdeckung ermöglicht es, dass das PAP-Gerät am Kopf des Patienten getragen und/oder gestützt werden kann.

US 4 257 415 A offenbart eine Vorrichtung zur Durchführung von Verneblerbehandlungen bei einem Patienten, auch während ambulanter Zeiten. Das Gerät, das tragbar und leicht ist, umfasst ein Gehäuse, das im Inneren mit einem batteriebetriebenen Kompressor versehen ist, der einen Vernebler mit Druckluft versorgt. Der Vernebler kann ein Medikament enthalten, das für die Inhalation durch einen Patienten über ein Mundstück oder eine Gesichtsmaske zerstäubt wird. Das Gerät ist so klein und leicht, dass es von einem Patienten während eines Spaziergangs getragen werden kann.

US 2012/145151 A1 offenbart eine Vorrichtung zum automatischen Zusammendrücken und Freigeben eines AMBU-Beutels. Die Vorrichtung hat einen Kompressor in dem Gehäuse. Im Gehäuse befinden sich Öffnungen, durch die die Einlass- und Auslassrohre des AMBU-Beutels in das Gehäuse hinein- und herausgeführt werden können. Ein elektrischer Antrieb treibt den Kompressor an. Alarme und Signale informieren über Überdruck und Sackwechsel.

US 2010/229867 A1 offenbart ein Beatmungsgerät umfassend einen Anzeigebildschirm zur Anzeige einer Vielzahl von Beatmungsgerätfunktionen/-modi oder Menüs und einer Vielzahl von Parametern oder Untermenüs, die mit mindestens einer der Beatmungsgerätfunktionen/-modi verbunden sind.

WO 2008/028247 A1 offenbart ein System zur Abgabe eines unter Druck stehenden Stroms von Atemgas an einen Patienten. Das System umfasst eine Patientenschnittstelle, die so konfiguriert ist, dass sie den Kopf des Patienten berührt.

US 2018/085541 A1 offenbart ein Beatmungsgerät umfassend ein Gebläse mit einem Einlass und einem Auslass, eine Patientenschnittstelle und ein Ventil mit einem Ventil Element, das durch eine erste Winkelverschiebung in einer ersten Richtung von einer ersten Position zu einer zweiten Position drehbar ist.

US 9 795 752 B2 offenbart ein kombiniertes Atemtherapie-Managementsystem, welches ein kombiniertes Atemtherapie-Rezept erstellt, das von einem kombinierten Atemtherapiegerät ausgeführt werden kann, um einem Patienten mehrere koordinierte Atemtherapien zuzuführen.

Demgegenüber ist es die Aufgabe der vorliegenden Erfindung, den Einsatz von Atemtherapiegeräten zu verbessern und insbesondere deren Benutzung und Bedienung auch bei einem eingeschränkten Raumangebot komfortabler zu gestalten. Insbesondere soll auch der Einsatz von zwei Atemtherapiegeräten, beispielsweise zur Beatmung und zur Hustenunterstützung, komfortabler gestaltet werden.

Diese Aufgabe wird erfindungsgemäß gelöst mit dem Atemtherapiegerät des Anspruchs 1 sowie mit dem System des Anspruchs 23 und mit dem System des Anspruchs 24. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Atemtherapiegerät dient zum Erzeugen einer Atemluftströmung für die Durchführung einer Atemtherapie. Das Atemtherapiegerät umfasst wenigstens eine Gehäuseeinrichtung zur Einhausung von Gerätekomponenten. Dabei umfasst die Gehäuseeinrichtung wenigstens zwei Aufstellflächen. Dadurch ist das Atemtherapiegerät in wenigstens zwei Aufstellpositionen bestimmungsgemäß aufstellbar und vorzugsweise bestimmungsgemäß betreibbar. Insbesondere wird das Atemtherapiegerät in den wenigstens zwei Aufstellpositionen bestimmungsgemäß betrieben. Das erfindungsgemäße Atemtherapiegerät bietet viele Vorteile. Einen erheblichen Vorteil bietet die Gehäuseeinrichtung mit den Aufstellflächen, sodass das Atemtherapiegerät in unterschiedlichen Aufstellpositionen aufgestellt und auch betrieben werden kann. So kann je nach Platzangebot und Umgebungsbedingungen die optimale Aufstellposition ausgesucht werden. Beispielsweise kann bei einer liegenden Person eine andere Aufstellposition als bei einer stehenden Person unaufwendig und zügig umgesetzt werden.

Vorzugsweise umfasst die Gehäuseeinrichtung wenigstens eine erste Aufstellfläche für wenigstens eine erste Aufstellposition. Vorzugsweise umfasst die Gehäuseeinrichtung wenigstens eine zweite Aufstellfläche für wenigstens eine zweite Aufstellposition. Insbesondere ist für jede Aufstellposition wenigstens eine Aufstellfläche vorgesehen. Die erste Aufstellfläche ist vorzugsweise für eine liegende Aufstellposition geeignet und ausgebildet. Die zweite Aufstellfläche ist vorzugsweise für eine stehende Aufstellposition geeignet und ausgebildet. In der liegenden Aufstellposition liegt das Atemtherapiegerät insbesondere auf der ersten Aufstellfläche. In der stehenden Aufstellposition steht das Atemtherapiegerät insbesondere auf der zweiten Aufstellfläche.

Solche Ausgestaltungen ermöglichen einen besonders einfachen und komfortablen Wechsel zwischen einer liegenden und einer stehenden Verwendung des Atemtherapiegeräts. Je nach Anforderungen an die Zugänglichkeit und je nach Aufstellort und Position des Benutzers kann das Gerät wahlweise stehend oder liegend verwendet werden. Für einen Wechsel zwischen den Aufstellpositionen muss es lediglich anders aufgestellt und beispielsweise von der stehenden in die liegende Position gekippt werden oder umgekehrt. So kann der Benutzer das Gerät ganz einfach dadurch an seine Bedürfnisse anpassen, indem er die Aufstellposition durch Aufrichten bzw. Kippen anpasst.

In der liegenden Aufstellposition ist eine Aufstellhöhe des Atemtherapiegeräts insbesondere geringer als eine Länge und/oder Breite der ersten Aufstellfläche. In der stehenden Aufstellposition ist eine Aufstellhöhe des Atemtherapiegeräts insbesondere größer als eine Länge und/oder Breite der zweiten Aufstellfläche. So können die Zugänglichkeit und der Raumbedarf des Geräts durch Wahl der Aufstellposition besonders vorteilhaft angepasst werden.

In der liegenden Aufstellposition entspricht die Aufstellhöhe insbesondere der Länge und/oder Breite der zweiten Aufstellfläche. In der stehenden Aufstellposition entspricht die Aufstellhöhe insbesondere der Länge und/oder Breite der ersten Aufstellfläche. Insbesondere ist die erste Aufstellfläche länger und/oder breiter als die zweite Aufstellfläche. Insbesondere sind die erste Aufstellfläche und die zweite Aufstellfläche gleich breit ausgebildet. Möglich ist auch, dass die erste Aufstellfläche und die zweite Aufstellfläche gleich lang ausgebildet sind. Insbesondere entsprechen die Aufstellflächen der Grundfläche des Atemtherapiegeräts in der Aufstellposition auf der jeweiligen Aufstellfläche.

Die liegende Aufstellposition ist insbesondere dadurch definiert, dass die maximale und/oder mittlere Höhe des Atemtherapiegeräts, insbesondere der Gehäuseeinrichtung, geringer als die maximale und/oder mittlere Länge und/oder maximale und/oder mittlere Breite des Atemtherapiegeräts, insbesondere der Gehäuseeinrichtung, ist. In der liegenden Aufstellposition ist die maximale und/oder mittlere Höhe des Atemtherapiegeräts, insbesondere der Gehäuseeinrichtung, insbesondere geringer als die maximale und/oder mittlere Länge und/oder maximale und/oder mittlere Breite der ersten Aufstellfläche, auf welcher das Atemtherapiegerät in der liegenden Aufstellposition aufgestellt ist.

Die stehende Aufstellposition ist insbesondere dadurch definiert, dass die maximale und/oder mittlere Höhe des Atemtherapiegeräts, insbesondere der Gehäuseeinrichtung, größer als die maximale und/oder mittlere Länge und/oder maximale und/oder mittlere Breite des Atemtherapiegeräts, insbesondere der Gehäuseeinrichtung, ist. In der stehenden Aufstellposition ist die maximale und/oder mittlere Höhe des Atemtherapiegeräts, insbesondere der Gehäuseeinrichtung, größer als die maximale und/oder mittlere Länge und/oder maximale und/oder mittlere Breite der zweiten Aufstellfläche, auf welcher das Atemtherapiegerät in der stehenden Aufstellposition aufgestellt ist.

Insbesondere sind die Aufstellflächen in einem Winkel von wenigstens 65° und maximal 115° und vorzugsweise in einem Winkel von wenigstens 80° und maximal 100° und besonders bevorzugt in einem Winkel von 90° +/-5° zueinander angeordnet.

Bevorzugt ist auch, dass die Aufstellflächen rechtwinklig zueinander angeordnet sind. Insbesondere ist die erste Aufstellfläche in einem der zuvor beschrienen Winkel zur zweiten Aufstellfläche angeordnet. In einer solchen Ausgestaltung kommen die Vorteile der verschiedenen Aufstellpositionen besonders gut zur Geltung. Möglich sind auch andere Winkel der Aufstellflächen zueinander.

Ein Geräteschwerpunkt des Atemtherapiegeräts ist erfindungsgemäß in einer unteren Gerätehälfte und vorzugsweise in einem unteren Viertel und besonders bevorzugt in einem unteren Achtel des Geräts angeordnet, wenigstens in derjenigen der wenigstens zwei Aufstellpositionen wie zuvor beschrieben, in welcher das Atemtherapiegerät eine größere Aufstellhöhe aufweist. Insbesondere ist der Geräteschwerpunkt wenigstens in der stehenden Aufstellposition derartig angeordnet. Insbesondere ist der Geräteschwerpunkt in derjenigen Aufstellposition derartig angeordnet, welche eine geringere Aufstellfläche als die andere Aufstellposition aufweist. Möglich ist auch, dass der Geräteschwerpunkt in allen bestimmungsgemäßen Aufstellpositionen derartig angeordnet ist. Insbesondere ist der Geräteschwerpunkt wenigstens in der stehenden Aufstellposition näher zur zweiten Aufstellfläche als zu einem oberen Geräteende angeordnet. Dadurch kann einem versehentlichen Umkippen des Geräts zuverlässig entgegengewirkt werden.

Der Geräteschwerpunkt wird erfindungsgemäß wenigstens durch die Anordnung wenigstens einer Akkueinrichtung zur Energieversorgung des Atemtherapiegeräts und/oder durch die Anordnung wenigstens einer Lüftereinrichtung zur Erzeugung einer Atemgasströmung bereitgestellt. Der Geräteschwerpunkt kann auch durch die Anordnung wenigstens einer anderen schweren Gerätekomponente bereitgestellt werden. Solche Ausgestaltungen begünstigen einen tiefen Schwerpunkt besonders in der stehenden Anordnung. Die Akkueinrichtung dient insbesondere zur Energieversorgung während einer Beatmung und/oder eines Hustenmanövers und stellt insbesondere die Energie für die Atemtherapie bereit.

Das Atemtherapiegerät umfasst insbesondere wenigstens eine Schnittstelle zum Koppeln von Schläuchen für eine Atemgasströmung und beispielsweise einen Beatmungsschlauch und/oder Hustenschlauch. Das Atemtherapiegerät umfasst insbesondere wenigstens einen Kabelanschluss für eine elektrische Energieversorgung und/oder zur Netzwerkanbindung oder dergleichen. Die Schnittstelle und/oder der Kabelanschluss können auch zum Anschließen von Zubehör für das Atemtherapiegerät geeigneten ausgebildet sein. Dabei sind die Schnittstelle und/oder der Kabelanschluss vorzugsweise wenigstens in derjenigen der wenigstens zwei Aufstellpositionen in einer unteren Gerätehälfte angeordnet, in welcher das Atemtherapiegerät eine größere Aufstellhöhe aufweist. Insbesondere sind die Schnittstelle und/oder der Kabelanschluss dabei in einem unteren Viertel und besonders bevorzugt in einem unteren Achtel des Geräts angeordnet. Eine solche Anordnung der Schnittstelle und/oder des Kabelanschlusses ist insbesondere in der stehenden Aufstellposition vorgesehen. Eine solche Anordnung der Schnittstelle und/oder des Kabelanschlusses kann auch in beiden Aufstellposition vorgesehen sein. Insbesondere sind die Schnittstelle und/oder der Kabelanschluss wenigstens in der stehenden Aufstellposition näher zur zweiten Aufstellfläche als zu einem oberen Geräteende angeordnet. So kann zuverlässig vermieden werden, dass bei einem versehentlichen Ziehen an einem Schlauch oder an einem Kabel zu große Drehmomente entstehen, durch welche das Gerät umkippen könnte.

Die Schnittstelle und/oder der Kabelanschluss sind in allen bestimmungsgemäßen Aufstellpositionen vorzugsweise seitlich an der Gehäuseeinrichtung angeordnet. Insbesondere befinden sich die Schnittstelle und der Kabelanschluss in allen bestimmungsgemäßen Aufstellpositionen, vorzugsweise wenigstens in der stehenden Aufstellposition, seitlich zu den Aufstellflächen. Dadurch kann einem Umstürzen durch versehentliches Ziehen besonders gut entgegengewirkt werden.

Das Atemtherapiegerät umfasst insbesondere wenigstens eine Griffeinrichtung. Die Griffeinrichtung dient insbesondere zum Tragen und/oder zum Verändern der Aufstellposition. Insbesondere ist die Griffeinrichtung derart angeordnet, dass die Griffeinrichtung in einer liegenden Aufstellposition an der Vorderseite und in einer stehenden Aufstellposition an einer Oberseite der Gehäuseeinrichtung befindlich ist. Dadurch ist die Griffeinrichtung insbesondere in beiden Aufstellposition von einer vor dem Atemtherapiegerät stehenden Person erreichbar. Insbesondere ist die Griffeinrichtung in beiden Aufstellpositionen von derselben Benutzerposition aus erreichbar. Insbesondere befindet sich die Griffeinrichtung in beiden Aufstellpositionen beabstandet zu der Ebene, auf welcher das Atemtherapiegerät aufgestellt ist. Das ermöglicht ein Umgreifen bzw. Ergreifen der Griffeinrichtung in allen Aufstellpositionen. Die Griffeinrichtung ist insbesondere in allen bestimmungsgemäßen Aufstellpositionen umgreifbar und/oder wenigstens ergreifbar.

Die Griffeinrichtung ist vorzugsweise zurückspringend an der Gehäuseeinrichtung angeordnet. Dadurch steht die Griffeinrichtung vorzugsweise nicht aus einem Konturverlauf der Gehäuseeinrichtung hervor. Insbesondere ist die Griffeinrichtung in die Gehäuseeinrichtung integriert und beispielsweise eingelassen. So wirkt der Griff in keiner der Aufstellpositionen hinderlich. Die Griffeinrichtung kann auch abstehend an der Gehäuseeinrichtung befestigt sein.

Die Griffeinrichtung ist vorzugsweise unbeweglich an der Gehäuseeinrichtung angeordnet. Insbesondere ist die Griffeinrichtung starr ausgebildet. Eine solche Ausgestaltung hat den Vorteil, dass das Gerät bewegt und die Aufstellposition verändert werden kann, ohne dass der Griff zuerst in eine bestimmte Position gebracht und beispielsweise herausgeklappt werden muss. Zudem erlaubt ein derart ausgestalteter Griff auf einfache und ergonomische Weise sowohl das Tragen des Gerätes als auch den schnellen Lagewechsel aus der liegenden in die stehende Aufstellposition und umgekehrt. Möglich ist aber auch, dass der Griff klappbar und/oder ausziehbar und/oder kippbar und/oder schwenkbar oder bewegbar ist.

Die Gehäuseeinrichtung umfasst insbesondere wenigstens eine Gehäusekante, welche als eine Kippkante ausgebildet ist oder wenigstens eine solche umfasst. Die Gehäuseeinrichtung wird insbesondere über die Kippkante gekippt, um zwischen den Aufstellpositionen zu wechseln. Dabei ist die Kippkante vorzugsweise nachgiebig und/oder verstärkt und insbesondere abriebverstärkt und/oder abgerundet ausgebildet. Möglich ist auch, dass die Kippkante auswechselbar und/oder gehärtet ausgebildet ist. Es ist möglich, dass die Kippkante wenigstens ein Schutzelement umfasst. Das Schutzelement ist insbesondere weich und/oder elastisch und/oder flexibel oder auch gehärtet und/oder auswechselbar ausgebildet. Eine solche Ausgestaltung hat den Vorteil, dass das Gerät gekippt werden kann, ohne vollständig angehoben werden zu müssen. Zudem erlaubt eine solche Kippkante den Kippvorgang bzw. Lagewechsel, ohne dass zum Beispiel Kratzer am Gerät oder an einer Aufstellfläche verursacht werden oder vom Anwender befürchtet werden müssen. Die Kippkante erstreckt sich insbesondere zwischen den beiden Aufstellflächen. Insbesondere laufen die beiden Aufstellflächen an der Kippkante zusammen bzw. aufeinander zu.

Solche Ausgestaltungen können auch für die Aufstellflächen wenigstens bereichsweise vorgesehen sein, um dort Abnutzung bzw. Beschädigung zu vermeiden. Möglich ist, dass die Aufstellflächen wenigstens teilweise nachgiebig und/oder verstärkt und insbesondere abriebverstärkt und/oder mit wenigstens einem Schutzelement ausgestattet sind.

Insbesondere ist die Griffeinrichtung gegenüberliegend von der Kippkante angeordnet. Dadurch wird insbesondere ein entsprechend großer Hebel erreicht, sodass das Gerät besonders einfach für einen Wechsel der Aufstellposition gekippt werden kann.

Die Aufstellflächen werden insbesondere durch jeweils wenigstens eine Seitenfläche der Gehäuseeinrichtung bereitgestellt. Insbesondere werden die Aufstellflächen durch jeweils eine gesamte Seitenfläche bereitgestellt. Das bietet besonders große Aufstellflächen, sodass eine besonders zuverlässige Kippstabilität erreicht wird. Möglich ist auch, dass die Aufstellflächen von wenigstens 75 % und insbesondere wenigstens 85 % und vorzugsweise wenigstens 90 % und besonders bevorzugt wenigstens 95 % der jeweiligen Seitenfläche bereitgestellt werden. Die Seitenfläche ist dabei insbesondere eine Außenfläche. Unter einer Seitenfläche wird insbesondere eine Vorderseite, Oberseite, Unterseite, Rückseite, rechte Seite und/oder linke Seite verstanden.

Besonders bevorzugt ist das Atemtherapiegerät dazu geeignet und ausgebildet, nur durch Aufstellen und vorzugsweise Kippen der Gehäuseeinrichtung und insbesondere ohne weitere Maßnahmen zwischen den Aufstellpositionen bestimmungsgemäß überführt zu werden. Insbesondere sind keine Verstellungen an der Gehäuseeinrichtung und/oder der Griffeinrichtung nötig. Insbesondere ist zum Überführen des Atemtherapiegeräts zwischen den Aufstellpositionen keine Kopplung und/oder Einstellung und/oder Veränderung von Bauteilen am Atemtherapiegerät nötig. Insbesondere müssen keine Schläuche und/oder Kabel oder dergleichen umgesteckt werden. Insbesondere kann das Atemtherapiegerät zwischen den Aufstellpositionen überführt werden, ohne dass das gesamte Atemtherapiegerät angehoben werden muss. Insbesondere ist das Atemtherapiegerät zwischen den Aufstellpositionen nur durch Kippen bzw. Verschwenken, insbesondere über die Kippkante, überführbar. Das bietet ein besonders komfortables Anpassen der Aufstellposition.

Es kann vorgesehen sein, dass die Atemtherapie während eines Betriebs, beispielsweise eine Beatmung und/oder eine Hustenunterstützung, ohne weitere Maßnahmen zwischen den Aufstellpositionen bestimmungsgemäß überführbar ist. Insbesondere muss dazu die Therapie nicht unterbrochen werden. Möglich ist auch, dass für die Überführung zwischen den Aufstellpositionen eine Therapiepause automatisch oder manuell eingestellt wird.

In einer besonders vorteilhaften Ausgestaltung ist das Atemtherapiegerät dazu geeignet und ausgebildet, mittels wenigstens einer Steuereinrichtung die eingenommene Aufstellposition selbstständig zu erkennen. Insbesondere ist das Atemtherapiegerät dazu geeignet und ausgebildet, mittels der Steuereinrichtung in Abhängigkeit der Aufstellposition wenigstens eine Geräteeinstellung zu verändern und/oder zu überwachen. Es ist möglich, dass der Steuereinrichtung zur Erkennung der Aufstellposition wenigstens eine Sensoreinrichtung zugeordnet ist. Zusätzlich oder alternativ kann mittels der Steuereinrichtung in Abhängigkeit der Aufstellposition wenigstens ein Benutzerhinweis ausgegeben werden, beispielsweise ein Hinweis für eine notwendige manuelle Anpassung eine Geräteeinstellung. Die Geräteeinstellung kann Gerätekomponenten des Atemtherapiegeräts und/oder Therapieeinstellungen für eine Beatmung oder für eine Hustentherapie umfassen.

Besonders bevorzugt ist das Atemtherapiegerät dazu geeignet und ausgebildet, mittels der Steuereinrichtung in Abhängigkeit der Aufstellposition wenigstens einen Darstellungsmodus einer Anzeigeeinrichtung und/oder wenigstens eine Funktionsbelegung einer Bedieneinrichtung einzustellen. Durch eine solche automatische Anpassung der Darstellung bzw. der Bedienung wird die flexible Aufstellung des Geräts noch komfortabler ausgestaltet. Insbesondere wird die Ausrichtung der Anzeigeeinrichtung und/oder der Bedieneinrichtung in Abhängigkeit der Aufstellposition so eingestellt, dass die Anzeige bzw. die Bedienelemente lesbar sind. Insbesondere wird die Ausrichtung der Anzeigeeinrichtung und/oder der Bedieneinrichtung gedreht und/oder gespiegelt. Die Bedieneinrichtung und/oder die Anzeigeeinrichtung können auch manuell über wenigstens eine Nutzereingabe gedreht werden.

Insbesondere umfasst das Atemtherapiegerät wenigstens eine Anzeigeeinrichtung und/oder wenigstens eine Bedieneinrichtung. Die Anzeigeeinrichtung ist insbesondere als ein Display ausgebildet oder umfasst wenigstens ein solches. Die Bedieneinrichtung kann wenigstens einen und vorzugsweise eine Mehrzahl von Hardkeys und/oder Softkeys umfassen. Insbesondere ist die Bedieneinrichtung als ein User-Interface ausgebildet oder umfasst wenigstens ein solches. Es ist möglich, dass die Bedieneinrichtung in die Anzeigeeinrichtung integriert ist. Beispielsweise kann eine Anzeigeeinrichtung mit einer berührungsempfindlichen Oberfläche, auch als Touchscreen bezeichnet, vorgesehen sein. Insbesondere erstreckt sich der Touchscreen dabei über die gesamte Anzeigeeinrichtung. Insbesondere ist eine kapazitiv arbeitende Bedieneinrichtung vorgesehen. Die Anzeigeeinrichtung kann als Bedien- und Anzeigeeinrichtung ausgebildet sein. Über eine solche berührungsempfindliche Oberfläche werden insbesondere eine Vielzahl von Softkeys bereitgestellt. Insbesondere ist die Ausrichtung und/oder Belegung der Softkeys in Abhängigkeit der Aufstellposition einstellbar. Zusätzlich oder alternativ kann wenigstens eine separat zur Anzeigeeinrichtung ausgebildete Bedieneinrichtung vorgesehen sein, welche vorzugsweise Hardkeys und z. B. Knöpfe oder Schalter umfasst. Die Hardkeys sind insbesondere an einer Seitenfläche der Gehäuseeinrichtung angeordnet, welche nicht bereits durch die Anzeigeeinrichtung und/oder eine Aufstellfläche belegt ist.

Die Anzeigeeinrichtung und/oder Bedieneinrichtung ist vorzugsweise an wenigstens einer Seitenfläche der Gehäuseeinrichtung angeordnet und insbesondere integriert. Dabei ist diese Seitenfläche vorzugsweise flächenmäßig die größte Seitenfläche der Gehäuseeinrichtung. Möglich und bevorzugt ist auch, dass die Anzeigeeinrichtung und/oder Bedieneinrichtung an wenigstens einer Seitenfläche der Gehäuseeinrichtung angeordnet ist, welche eine größere Fläche als die einzelnen Flächen der Aufstellflächen aufweist. So können die Informationen auf dem Display besonders gut erkannt und übersichtlich dargestellt werden.

Die Seitenfläche mit der Anzeigeeinrichtung und/oder Bedieneinrichtung ist insbesondere wenigstens 1,5-mal und vorzugsweise zweimal größer als die Fläche der zweiten Aufstellfläche. Die Seitenfläche mit der Anzeigeeinrichtung und/oder Bedieneinrichtung ist insbesondere wenigstens 1,1-mal und vorzugsweise wenigstens 1,3-mal größer als die Fläche der ersten Aufstellfläche. Die Seitenfläche mit der Anzeigeeinrichtung und/oder Bedieneinrichtung kann wenigstens genauso groß sein wie die Fläche der ersten Aufstellfläche. Die Anzeigeeinrichtungen oder Bedieneinrichtung können auch an einer kleineren Seitenfläche angeordnet sein. Es können auch Anzeigeeinrichtungen und/oder Bedieneinrichtungen an wenigstens einer weiteren Seitenfläche vorgesehen sein.

In einer besonders vorteilhaften Ausgestaltung ist vorgesehen, dass die Anzeigeeinrichtung wenigstens 70 % und insbesondere wenigstens 95 % der Seitenfläche der Gehäuseeinrichtung einnimmt. Es ist möglich und bevorzugt, dass die Anzeigeeinrichtung bis zu 100 % der Seitenfläche einnimmt. Möglich ist auch, dass die Anzeigeeinrichtung sich über die Seitenfläche, an welcher sie angeordnet ist, hinaus auf wenigstens eine benachbarte Seitenfläche erstreckt. Dabei beziehen sich die vorliegend genannten Angaben über die Anordnung und Größe der Anzeigeeinrichtung insbesondere auf deren sichtbare Fläche bzw. Displayfläche.

In einer besonders vorteilhaften Weiterbildung ist die Bedieneinrichtung wenigstens in einem oberen Viertel und vorzugsweise wenigstens in einem oberen Achtel mit geringeren Kräften betätigbar, als die zum Kippen der Gehäuseeinrichtung notwendigen Kräfte. Dabei ist die Bedieneinrichtung insbesondere in die Anzeigeeinrichtung integriert und mit dieser als eine berührungsempfindliche Oberfläche bzw. Touchscreen ausgebildet. Insbesondere umfasst die Bedieneinrichtung dazu Softkeys. Bevorzugt sind die zuvor beschriebenen Bedienkräfte auch bei einer Bedieneinrichtung mit Hardkeys vorgesehen. Es ist möglich, dass auch außerhalb der Anzeigeeinrichtung Hardkeys angeordnet sind, welche mit den zuvor beschriebenen Bedienkräften betätigbar sind. Dabei ist vorzugsweise auch der Schwerpunkt wie zuvor beschrieben ausgebildet, sodass die zum Kippen der Gehäuseeinrichtung notwendigen Kräfte entsprechend groß sind. So ist eine zuverlässige Bedienung des Geräts auch in der stehenden Position möglich, ohne dass ein Kippen bedingt durch Bedienkräfte zu befürchten ist.

In allen Ausgestaltungen ist es bevorzugt, dass die Anzeigeeinrichtung gegenüber der ersten Aufstellfläche zwischen 0° und 55° und besonders bevorzugt 10° und 45° geneigt ist. Es ist ebenfalls in allen Ausgestaltungen bevorzugt, dass die Anzeigeeinrichtung gegenüber der zweiten Aufstellfläche zwischen 40° und 95° und besonders bevorzugt zwischen 45° und 85° geneigt ist. In der stehenden Aufstellposition ist die Anzeigeeinrichtung insbesondere 10° bis 45° zur vertikalen geneigt. In der liegenden Aufstellposition ist die Anzeigeeinrichtung insbesondere 45° bis 85° zur vertikalen geneigt. Möglich sind auch andere Winkelpositionen der Anzeigeeinrichtungen. Möglich ist auch, dass die Anzeigeeinrichtung im Winkel verstellbar ist. Vorzugsweise ist ein Betrachtungswinkel der Anzeigeeinrichtung in der liegenden Aufstellposition für eine stehende Person angepasst bzw. optimiert. Vorzugsweise ist ein Betrachtungswinkel der Anzeigeeinrichtung in der stehenden Aufstellposition für eine liegende bzw. sitzende Person angepasst. Dadurch muss das Gerät lediglich gekippt bzw. hingelegt werden, wenn sich der Benutzer ins Bett legt oder hinsetzt. In einer Ausgestaltung ist in dem Atemtherapiegerät auf mindestens einer der flächenmäßig kleineren Seitenflächen zusätzlich mindestens ein weiteres Bedienelement angeordnet.

Bevorzugt befinden sich mindestens ein oder zwei weitere Bedienelemente auf der flächenmäßig kleineren Seitenfläche, in der auch die Griffeinrichtung angeordnet sein kann. Wiederum bevorzugt ist je ein Bedienelement auf je einer der beiden Seiten der Griffeinrichtung angeordnet, wobei besondere Funktionen mit diesen Bedienelementen gesteuert werden. Dies können beispielsweise die Ein/Ausschaltung von Gerät, Therapiestart/Stopp und oder die Bestätigung bzw. Stummschaltung von Alarmen sein. Durch die Anordnung dieser speziellen Funktionen - je nach gewählter Lage des Gerätes - auf einer dem Anwender besonders zugewandten Seitenfläche oder einer im Wesentlichen nach oben gerichteten Seitenfläche befinden sich diese Funktionen in einem besonders direkten und gut wahrnehmbaren Zugriff durch den Anwender.

In allen Ausgestaltungen ist das Atemtherapiegerät dazu geeignet und ausgebildet, eine betriebsgemäß erforderliche Atemluftströmung unabhängig von der Aufstellposition bereitzustellen. Insbesondere ist in allen bestimmungsgemäßen Aufstellpositionen die betriebsgemäß erforderliche Atemluftströmung bereitstellbar. Insbesondere sind alle betriebsgemäß vorgesehenen Therapieformen in allen bestimmungsgemäßen Aufstellpositionen durchführbar. Dabei wird die Atemluftströmung insbesondere von der Lüftereinrichtung bereitgestellt. Insbesondere ist wenigstens ein Ansaugbereich der Lüftereinrichtung derart ausgebildet, dass eine Atemluftansaugung in allen bestimmungsgemäßen Aufstellpositionen funktioniert und insbesondere keine Gefahr einer Verblockung entsteht.

Es ist bevorzugt, dass alle bestimmungsgemäß vorgesehenen Öffnungen der Gehäuseeinrichtung in allen bestimmungsgemäßen Aufstellpositionen wenigstens gegen feste Fremdkörper mit einem Durchmesser größer oder gleich 12,5 mm und/oder gegen den Zugang mit einem Finger geschützt sind und/oder einen Schutz gegen fallendes Tropfwasser, wenn die Gehäuseeinrichtung bis zu 15° geneigt ist, aufweisen. Insbesondere erfüllt das Gerät bzw. die Gehäuseeinrichtung wenigstens die am Anmeldetag gültige Schutzklasse IP22 oder eine höhere Schutzklasse.

Das Atemtherapiegerät ist besonders bevorzugt als ein tragbares Atemtherapiegerät ausgebildet. Insbesondere weist das Atemtherapiegerät ein maximales Gewicht von 10 kg und besonders bevorzugt ein maximales Gewicht von 6 kg auf. Insbesondere ist das Atemtherapiegerät mittels wenigstens einer Griffeinrichtung tragbar. Insbesondere weist das Atemtherapiegerät eine netzunabhängige Energieversorgung mittels wenigstens einer Akkueinrichtung auf. Insbesondere weist das Atemtherapiegerät ein Gewicht zwischen 1 kg und 10 kg und bevorzugt zwischen 1,5 kg und 6 kg auf. In einer solchen Ausgestaltung kommen die Vorteile der verschiedenen Aufstellpositionen besonders gut zur Geltung. Möglich ist auch, dass das Atemtherapiegerät als ein stationäres Gerät ausgebildet ist.

Die Gehäuseeinrichtung ist vorzugsweise dazu geeignet und ausgebildet, in wenigstens einer der Aufstellpositionen mit einer Gehäuseeinrichtung eines weiteren erfindungsgemäßen Atemtherapiegeräts in einer sich gegenseitig abstützenden Anordnung, auch als Rücken-an-Rücken-Anordnung bezeichnet, aufgestellt und betrieben zu werden. Vorzugsweise ergänzen sich die Aufstellflächen der jeweiligen Gehäuseeinrichtung dabei zu einer gemeinsamen Aufstellfläche. Dabei stützt sich die erste Aufstellfläche insbesondere an der ersten Aufstellfläche der anderen Gehäuseeinrichtung ab. Die Gehäuseeinrichtungen stehen dabei vorzugsweise mit den für die liegende Aufstellposition vorgesehenen Aufstellflächen aneinander. Insbesondere können sich die Atemtherapiegeräte dabei gemeinsam abstützen. Das bietet eine besonders stabile und kippsichere Anordnung der Atemtherapiegeräte. Insbesondere befinden sich die Gehäuseeinrichtung dabei in der gleichen Aufstellposition, beispielsweise beide stehend oder beide liegend.

Besonders bevorzugt ist eine sich gegenseitig abstützende Anordnung in der stehenden Aufstellposition vorgesehen. Die sich gegenseitig abstützende Anordnung kann auch in der liegenden Aufstellposition vorgesehen sein. Möglich ist aber auch, dass bei der sich gegenseitig abstützenden Anordnung ein Gerät in der stehenden und das andere Gerät in der liegenden Aufstellposition ist. Es ist möglich, dass sich die Atemtherapiegeräte bei einer sich gegenseitig abstützenden Anordnung in einer Wirkverbindung stehen. Beispielsweise können in einer solchen Anordnung die Steuereinrichtungen drahtlos und/oder drahtgebunden miteinander gekoppelt sein.

Es wird ein System beschrieben, welches aus wenigstens zwei Atemtherapiegeräten besteht, wie sie zuvor beschrieben wurden. Dabei sind die beiden Atemtherapiegeräte mittels ihrer Gehäuseeinrichtung in der sich gegenseitig abstützenden Anordnung aufstellbar und vorzugsweise betreibbar. Es ist möglich, dass die beiden Atemtherapiegeräte in dieser Anordnung automatisch eine Wirkverbindung miteinander herstellen. Insbesondere ist dabei ein Atemtherapiegerät als Hustengerät und das andere Atemtherapiegerät als Beatmungsgerät ausgebildet oder betreibbar.

Besonders bevorzugt ist die Gehäuseeinrichtung dazu geeignet und ausgebildet, wenigstens in der stehenden Aufstellposition an einem Rollstuhl und/oder an einem Krankenbett befestigt zu werden. Insbesondere begünstigt die Gehäuseeinrichtung eine solche Anordnung. Besonders bevorzugt ist die Gehäuseeinrichtung in der stehenden Aufstellposition hinten an einem Rollstuhl befestigt war. Dazu können die entsprechenden Aufstellflächen mit wenigstens einer Montageeinrichtung ausgestattet sein.

Das Atemtherapiegerät ist vorzugsweise als ein Hustengerät ausgebildet. Das Atemtherapiegerät kann bevorzugt auch als ein Beatmungsgerät ausgebildet sein. Insbesondere ist das Atemtherapiegerät dazu geeignet und ausgebildet, sowohl als Hustengerät als auch als Beatmungsgerät eingesetzt zu werden. In einer solchen Ausgestaltung kann die Erfindung besonders vorteilhaft eingesetzt werden.

Ein System gemäß einem Aspekt der Erfindung umfasst wenigstens zwei erfindungsgemäße Atemtherapiegeräte und eine Koppeleinrichtung zur Kopplung von den wenigstens zwei Atemtherapiegeräten. Die Koppeleinrichtung umfasst erfindungsgemäß wenigstens zwei Koppeleinheiten, welche über ein Koppelelement miteinander verbindbar sind. Dabei ist erfindungsgemäß an wenigstens einer Koppeleinheit das eine Atemtherapiegerät mittels dessen Gehäuseeinrichtung liegend aufnehmbar. Dabei ist erfindungsgemäß an wenigstens einer anderen Koppeleinheit das andere Atemtherapiegerät mittels dessen Gehäuseeinrichtung stehend aufnehmbar. Dadurch sind die Atemtherapiegeräte in unterschiedlichen Aufstellpositionen, also zum Beispiel eines liegend und eines stehend, gleichzeitig an der Koppeleinrichtung aufnehmbar und insbesondere daran bestimmungsgemäß betreibbar.

Eine solche Koppeleinrichtung ermöglicht einen besonders komfortablen und gut erreichbaren und zugleich übersichtlichen Betrieb der zwei Atemtherapiegeräte. So sind die Geräte durch die stehende und liegende Anordnung besonders platzsparend aufstellbar. Besonders vorteilhaft ist eine solche Koppeleinrichtung beispielsweise dann, wenn ein Patient mit einem Hustengerät und zugleich mit einem Beatmungsgerät versorgt werden muss. Zudem kommen die Vorteile des erfindungsgemäßen Atemtherapiegeräts in Kombination mit der hierin beschriebenen Koppeleinrichtung besonders gut zur Geltung. Insbesondere sind die Aufstellflächen der Gehäuseeinrichtungen dazu geeignet und ausgebildet, mit der Koppeleinrichtung gekoppelt zu werden bzw. an der Koppeleinrichtung befestigt zu werden. Insbesondere erfolgen sowohl die liegende Kopplung als auch die stehende Kopplung des jeweiligen Atemtherapiegeräts über dessen erste Aufstellfläche. Insbesondere ist die erste Aufstellfläche dazu geeignet und ausgebildet, sowohl mit der ersten als auch mit der zweiten Koppeleinheit gekoppelt zu werden. Insbesondere wird das stehende Atemtherapiegerät mit der ersten Aufstellfläche an die Koppeleinrichtung gekoppelt und insbesondere wird auch das liegende Atemtherapiegerät mit seiner ersten Aufstellfläche an die Koppeleinrichtung gekoppelt. Möglich ist, dass zusätzlich oder alternativ eine Kopplung mit der zweiten Aufstellfläche an die Koppeleinrichtung vorgesehen ist. Es ist möglich, dass die liegende Aufnahme über die erste und/oder zweite Aufstellfläche erfolgt. Es ist auch möglich, dass die stehende Aufnahme über die erste und/oder zweite Aufstellfläche erfolgt. Die Koppeleinheiten umfassen insbesondere jeweils wenigstens ein Koppelschwert und jeweils wenigstens eine Koppelplatte. Dabei sind die Koppelschwerter insbesondere an dem Koppelelement befestigt. Die Koppelplatten sind insbesondere an den Gehäuseeinrichtungen der Atemtherapiegeräte befestigbar. Dabei ist das Koppelschwert zur Befestigung des Atemtherapiegeräts an der Koppeleinrichtung vorzugsweise in die Koppelplatte einschiebbar. Die Verbindung von Koppelschwert und Koppelplatte ist insbesondere werkzeuglos lösbar und vorzugsweise durch wenigstens eine Sicherungseinrichtung gesichert bzw. arretierbar, beispielsweise durch Verrasten oder dergleichen. Möglich ist auch eine umgekehrte Anordnung, bei der die Koppelschwerter an den Gehäuseeinrichtungen der Atemtherapiegeräte und die Koppelplatten an dem Koppelelement befestigt sind. Unter einem Koppelschwert bzw. einer Koppelplatte wird jede Geometrie verstanden, welche eine geeignete und lösbare Verbindung ermöglicht.

Die Koppelplatte bzw. das Koppelschwert können von der Koppeleinrichtung oder auch vom Atemtherapiegerät bereitgestellt werden. So kann die Koppelplatte zum Beispiel fest am Atemtherapiegerät montiert sein oder in die Gehäuseeinrichtung integriert sein. Möglich ist aber auch, dass die Koppelplatte ein Zubehör ist, welches an der Gehäuseeinrichtung nachgerüstet werden kann. Das Koppelschwert kann fest oder auch lösbar mit dem Koppelelement verbunden sein.

Es ist möglich, dass wenigstens eine Koppeleinheit, vorzugsweise diejenige für die Kopplung des liegenden Atemtherapiegeräts, mit wenigstens einem Zusatzgriff ausgestattet ist. Der Zusatzgriff dient insbesondere zum Bewegen der Koppeleinrichtung mit einem oder zwei Atemtherapiegeräten. Beispielsweise kann die Koppeleinrichtung dazu mit Rollen oder Rädern ausgestattet werden, um das gesamte System über den Zusatzgriff komfortabel verschieben zu können. Die Koppeleinrichtung ist insbesondere an einem Rollstuhl und/oder an einem Krankenbett und/oder an einer anderen mobilen oder stationären Einrichtung befestigbar. Die Koppeleinrichtung kann auch dazu geeignet und ausgebildet sein, auf einer Unterlage und beispielsweise einem Tisch oder einem Schrank abgestellt zu werden.

Es ist möglich, dass die Atemtherapiegeräte mittels wenigstens einer Steuereinrichtung dazu geeignet und ausgebildet sind, eine Kopplung an die Koppeleinrichtung automatisch zu erkennen. Insbesondere ist mittels der Steuereinrichtung in Abhängigkeit der erkannten Kopplung wenigstens eine Geräteeinstellung veränderbar. Beispielsweise kann die Bedieneinrichtung und/oder Anzeigeeinrichtung ausrichtbar sein. Möglich ist auch, dass in Abhängigkeit der Kopplung eines der Atemtherapiegeräte als Hustengerät und das andere Atemtherapiegerät als Beatmungsgerät ausgewählt und betrieben wird. Es ist möglich, dass sich die über die Koppeleinrichtung gekoppelten Atemtherapiegeräte wenigstens teilweise in ihrem Betrieb ergänzen. Beispielsweise kann eine Erweiterung der Anzeigeeinrichtung über beide Atemtherapiegeräte erfolgen. Die über die Koppeleinrichtung gekoppelten Atemtherapiegeräte können mittels der Steuereinrichtung in einer Wirkverbindung stehen oder auch ohne Wirkverbindung betrieben werden.

Das Koppelelement ist insbesondere als wenigstens ein Rohrelement ausgebildet oder umfasst wenigstens ein solches. Insbesondere sind die Atemtherapiegeräte mittels der Koppeleinheiten an unterschiedlichen Längsseiten des Koppelelements und vorzugsweise des Rohrelements befestigbar. Das Rohrelement ist insbesondere als ein Mehrkantrohr und vorzugsweise ein Vierkantrohr ausgebildet. Das Koppelelement kann ein Profilelement sein oder ein solches umfassen. Das Rohrelement weist insbesondere einen Hohlraum auf, der als Aufnahme beispielsweise für Atemtherapiezubehör oder Verbrauchsmaterial ausgebildet ist.

Es ist möglich, dass das Koppelelement mit wenigstens einem längsseitlichen Fortsatz ausgestattet ist. An dem Fortsatz ist insbesondere die Koppeleinheit des liegenden Atemtherapiegeräts angeordnet.

Die Koppeleinheiten sind insbesondere in einem Winkel von 65° bis 115° und vorzugsweise in einem Winkel von 80° bis 100° und besonders bevorzugt rechtwinklig zueinander angeordnet. Insbesondere sind die Koppeleinheiten derart angeordnet, wenn die beiden Atemtherapiegeräte bestimmungsgemäß an die Koppeleinrichtung gekoppelt sind. Insbesondere sind die Koppeleinheiten an dem Koppelelement und vorzugsweise an dem Rohrelement derartig angeordnet. Insbesondere sind die Koppelschwerter und/oder Koppelplatten derartig angeordnet.

Ein System nach einem witeren Aspekt der Erfindung besteht aus wenigstens zwei erfindungsgemäßen Atemtherapiegeräten und einer Koppeleinrichtung zur Kopplung von den wenigstens zwei Atemtherapiegeräten, wobei das eine Atemtherapiegerät als Beatmungsgerät und das andere als Hustengerät ausgebildet ist. Auch ein solches System löst die zuvor genannte Aufgabe besonders vorteilhaft und bietet eine besonders komfortable und bedienungsfreundliche Benutzung von Atemtherapiegeräten.

Die Aufstellflächen sind insbesondere eben bzw. planar ausgebildet. Dabei können die Aufstellflächen bereichsweise auch Erhebungen und/oder Vertiefungen aufweisen. Beispielsweise können die Aufstellflächen mit Füßen und/oder Sockeln oder dergleichen ausgestattet sein. Die Aufstellflächen sind insbesondere außen an der Gehäuseeinrichtung angeordnet. Unter der Aufstellfläche wird erfindungsgemäß die Fläche verstanden, welche nach unten zum Aufstellort gerichtet ist. Insbesondere erfolgt ein Aufstellen des Atemtherapiegeräts dadurch, dass die Aufstellflächen auf einer Unterlage und beispielsweise einem Tisch oder einem Boden oder einer Ablagefläche oder dergleichen aufgesetzt werden. Die Aufstellflächen sind insbesondere benachbart an der Gehäuseeinrichtung angeordnet. Die Aufstellflächen können durch benachbarte Seitenflächen oder auch durch Seitenflächen, welche von wenigstens einer weiteren Seitenfläche beabstandet sind, bereitgestellt werden. Die Aufstellfläche ist insbesondere in die Gehäuseeinrichtung integriert und/oder einstückig mit der Gehäuseeinrichtung verbunden. Möglich ist auch, dass die Aufstellfläche separat zur Gehäuseeinrichtung ausgebildet ist und beispielsweise an dieser lösbar oder unlösbar befestigt ist.

Das Atemtherapiegerät umfasst insbesondere wenigstens eine elektrisch betreibbare bzw. betriebene Atemgasquelle. Insbesondere umfasst die Atemgasquelle wenigstens eine Lüftereinrichtung und/oder Gebläseeinrichtung oder ist als eine solche ausgebildet. Die Atemgasquelle ist insbesondere von der Gehäuseeinrichtung eingehaust. Das Atemtherapiegerät ist insbesondere elektrisch betreibbar. Es kann ein stromnetzgebundener und/oder akkubetriebener Betrieb vorgesehen sein.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Systems mit einer Koppeleinrichtung mit zwei erfindungsgemäßen Atemtherapiegeräten in einer perspektivischen Ansicht;
- Fig. 2: das System der Fig. 1 in einer Ansicht von hinten;
- Fug. 3: ein erfindungsgemäßes Atemtherapiegerät in einer perspektivischen Ansicht;
- Fig. 4: eine Koppeleinrichtung in einer perspektivischen Ansicht;
- Fig. 5: die Koppeleinrichtung der Fig. 4 in einer Ansicht von schräg unten;
- Fig. 6: eine perspektivische Detaildarstellung der Koppeleinrichtung der Fig. 4 in einer Ansicht von schräg oben;
- Fig. 7: eine andere perspektivische Detaildarstellung der Koppeleinrichtung der Fig. 4 in einer Ansicht von schräg oben; und
- Fig. 8: eine perspektivische Detaildarstellung der Koppeleinrichtung der Fig. 4 in einer Ansicht von schräg unten.

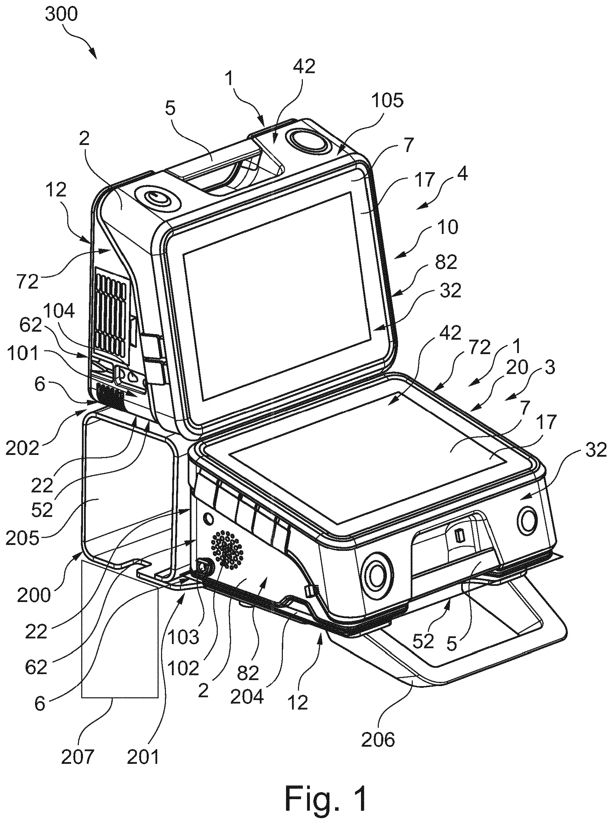

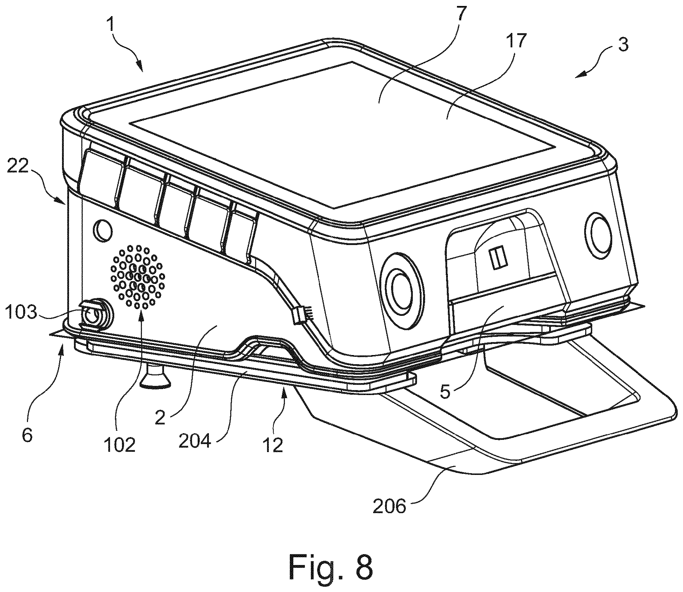

Die Figur 1 zeigt zwei erfindungsgemäße Atemtherapiegeräte 1, welche hier über eine erfindungsgemäße Koppeleinrichtung 200 zu einem System 300 gekoppelt sind. Ein solches System 300 bietet eine besonders bedienungsfreundliche und Raum sparende Anordnung von zwei Atemtherapiegeräten 1. Die Atemtherapiegeräte 1 können auch einzeln bzw. alleine (stand alone) ohne die Koppeleinrichtung 200 verwendet werden. Ein einzelnes erfindungsgemäßes Atemtherapiegerät 1 ist in der Figur 8 gezeigt.

Das eine Atemtherapiegerät 1 ist hier als Beatmungsgerät 20 und das andere als Hustengerät 10 ausgebildet. Das Beatmungsgerät 20 umfasst hier eine nicht sichtbar im Inneren angeordnete Lüftereinrichtung 102, um eine Atemluftströmung zur Beatmung des Patienten zu erzeugen. Das Hustengerät 10 dient zur gezielten Unterstützung einer Sekretabfuhr aus den Atemwegen eines Patienten und nutzt die Lüftereinrichtung 102 zur Insufflation und Exsufflation.

Die innenliegenden Gerätekomponenten des Atemtherapiegeräts 1 sind hier durch eine Gehäuseeinrichtung 2 eingehaust. Die Gehäuseeinrichtung 2 umfasst hier mehrere Seitenflächen, nämlich eine Vorderseite 32, Oberseite 42, Unterseite 52, Rückseite 62, und zwei seitlich angeordnete Seiten 72, 82. Das Gerät 1 ist hier als tragbares Atemtherapiegerät mit einem Gewicht von maximal 6 kg ausgestattet.

Zudem umfasst das Atemtherapiegerät 1 hier eine Anzeigeeinrichtung 7 und eine Bedieneinrichtung 17. Dabei wird ein Teil der Bedieneinrichtung 17 durch eine berührungsempfindliche Oberfläche der Anzeigeeinrichtung 7 bereitgestellt. Zudem umfasst die Bedieneinrichtung 17 verschiedene Hardkeys, beispielsweise Knöpfe und Schalter.

Das Atemtherapiegerät 1 kann in zwei verschiedenen Aufstellpositionen 3, 4 aufgestellt und betrieben werden. Das untere Atemtherapiegerät 1 des Systems 300 befindet sich hier in einer liegenden Aufstellposition 3 und das obere Atemtherapiegerät 1 in einer stehenden Aufstellposition 4. Um diese Aufstellpositionen 3, 4 zu ermöglichen, umfasst die Gehäuseeinrichtung 2 hier zwei Aufstellflächen 12, 22. Dabei ist eine erste Aufstellfläche 12 für die liegende Aufstellposition 3 und eine zweite Aufstellfläche 22 für die stehende Aufstellposition 4 vorgesehen.

Bei dem liegenden Gerät 1 entspricht die erste Aufstellfläche der Unterseite 52 und die zweite Aufstellfläche 22 der Rückseite 62. Bei dem stehenden Gerät 1 entspricht die erste Aufstellfläche 12 der Rückseite 62 und die zweite Aufstellfläche 22 der Unterseite 52.

Um das einzelne Atemtherapiegerät 1 ohne die Koppeleinrichtung 200 von der liegenden in die stehende Aufstellposition 3, 4 oder umgekehrt zu bringen, muss es lediglich gekippt werden. Dabei ist der Wechsel zwischen den Aufstellpositionen 3, 4 ohne weitere Maßnahmen möglich, wie zum Beispiel anzukoppelnde oder ausklappbare Elemente. Das wird hier unter anderem dadurch erreicht, dass die Aufstellflächen 12, 22 hinreichend groß ausgestaltet sind und dass ein Geräteschwerpunkt auch für die stehende Lage ausreichend tief liegt. Zudem ist für den Lagewechsel auch kein Umstecken von Schläuchen oder Kabeln notwendig. Des Weiteren kann das Atemtherapiegerät 1 die für die jeweilige Therapie erforderliche Atemluftströmung unabhängig von der Aufstellposition 3, 4 bereitstellen. Zudem sind die zum Ansaugen der Luft vorgesehenen Öffnungen so angeordnet, dass in allen Aufstellpositionen 3, 4 eine Atemluftansaugung zuverlässig ermöglicht wird und keine Gefahr der Verblockung entsteht. Die zum Ansaugen notwendigen Öffnungen sowie auch alle weiteren Öffnungen der Gehäuseeinrichtung 2 entsprechen hier der Schutzklasse IP22.

Der Geräteschwerpunkt ist hier in der stehenden Aufstellposition 4 in einer unteren Gerätehälfte angeordnet. Dazu wurden hier eine Akkueinrichtung 101 zur Energieversorgung sowie auch die Lüftereinrichtung 102 in der unteren Gerätehälfte angeordnet. Zudem wurden die Schnittstelle 103 zum Koppeln von Beatmungsschläuchen sowie die Kabelanschlüsse 104 in der unteren Gerätehälfte angeordnet. So bleibt das Gerät 1 auch bei einem versehentlichen Ziehen an einem Schlauch oder Kabel stabil in der entsprechenden Aufstellposition 3, **4.** Die Schnittstellen 103 und die Kabelanschlüsse 104 wurden zudem so angeordnet, dass sie sich in allen Aufstellposition 3, 4 seitlich an der Gehäuseeinrichtung 2 befinden.

Um einen möglichst unaufwendigen und komfortablen Wechsel zwischen den Aufstellpositionen 3, 4 zu ermöglichen, ist hier eine integrierte Griffeinrichtung 5 vorgesehen, welche zurückspringend an der Gehäuseeinrichtung 2 angeordnet ist. Dadurch steht die Griffeinrichtung 5 nicht aus dem Konturverlauf der Gehäuseeinrichtung 2 hervor. Zudem ist der Griff so ausgebildet und angeordnet, dass er in beiden Aufstellpositionen 3, 4 umgriffen werden kann. Zudem ist die Griffeinrichtung 5 starr ausgebildet und muss daher vor dem Ändern der Aufstellposition 3, 4 nicht erst umgeklappt oder herausgeklappt werden. So erlaubt die Griffeinrichtung 5 auf einfache und ergonomische Weise sowohl das Tragen des Geräts 1 als auch den schnellen Lagewechsel aus der liegenden in die stehende Position 3, 4 und umgekehrt. Zudem ist die Griffeinrichtung 5 4 in beiden Aufstellpositionen 3, 4 von einer vor dem Atemtherapiegerät 1 stehenden Benutzerperson erreichbar.

Um das Aufstellen bzw. Hinlegen des Geräts 1 weiter zu vereinfachen, ist die Gehäuseeinrichtung 2 hier mit einer als Kippkante 6 ausgebildeten Gehäusekante ausgestattet. Dadurch kann das Gerät 1 für den Lagewechsel über die Unterkante bzw. Hinterkante gekippt werden, ohne dass die Benutzerperson das Gerät 1 hierzu vollständig angeben muss. Dabei ist die Kippkante 6 vorzugsweise auf die beiden Aufstellflächen 12, 22, mit geeigneten schützenden, weichen oder nachgiebigen Aufstellerelementen versehen, die den Kippvorgang und Lagewechsel erlauben, ohne dass zum Beispiel Kratzer am Gerät oder an eine Aufstellfläche 12, 22 verursacht werden oder vom Anwender befürchtet werden müssen.

Das Atemtherapiegerät 1 umfasst hier eine Steuereinrichtung 105, welche die jeweilige Aufstellposition 3, 4 beispielsweise mittels einer Sensoreinrichtung selbstständig erkennt und **z. B.** daraufhin eine Geräteeinstellung verändert. Beispielsweise wird in Abhängigkeit der Aufstellposition 3, 4 die Ausrichtung des Touchscreens entsprechend gedreht bzw. gespiegelt.

Die Anzeigeeinrichtungen 7 weist hier eine Darstellungsfläche auf, welche im Wesentlichen die gesamte Fläche der größten Seitenfläche des Geräts 1 abdeckt. Das entspricht hier in der liegenden Position 3 der Oberseite 42 und in der stehenden Position 4 der Vorderseite 32. Dabei ist der Betrachtungswinkel der Anzeigeeinrichtung 7 hier in der liegenden Aufstellposition 3 für eine stehende Person optimiert. In der stehenden Aufstellposition 4 ist der Betrachtungswinkel dann entsprechend für eine liegende bzw. sitzende Person optimiert. Die hier gezeigten Atemtherapiegeräte 1 können auch ohne die Koppeleinrichtung 2 in einer Rücken-an-Rücken-Anordnung aufgestellt und betrieben werden. Dazu werden beide Geräte 1 in der stehenden Position 4 aufgestellt, sodass sie auf den zweiten Aufstellflächen 22 stehen. Die ersten Aufstellflächen 12, also die Rückseiten 62, berühren sich dann, sodass sich die Geräte stets oder im Falle eines Anstoßens gegenseitig abstützen können.

Die Koppeleinrichtung 200 ermöglicht eine mechanische und zuverlässige Verbindung der beiden Atemtherapiegeräte **1.** Dazu ist das liegende Atemtherapiegerät 1 über eine erste Koppeleinheit 201 und das stehende Atemtherapiegerät 1 über eine zweite Koppeleinheit 202 an ein Koppelelement angebunden. Das Koppelelement 205 ist hier als ein Rohrelement und beispielsweise als ein Vierkantrohr ausgebildet.

An das Koppelelement 205 kann in einer Ausgestaltung ein hier stark schematisiert dargestelltes Fahrgestell 207 montiert werden. Dadurch kann das gesamte System 300 besonders einfach bewegt und flexibel gehandhabt werden. Zum Ergreifen des Systems 300 ist dann beispielsweise ein Zusatzgriff 206 vorgesehen, welcher in der hier gezeigten Ausgestaltung im Bereich des liegenden Atemtherapiegeräts 1 angeordnet ist.

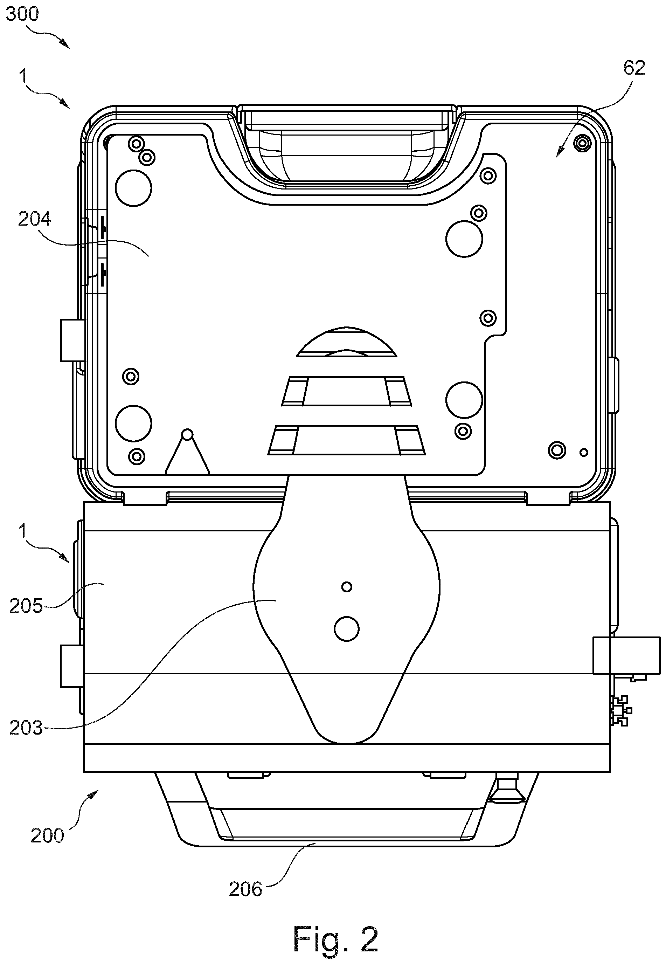

Die Koppeleinrichtung 200 bzw. das System 300 werden nachfolgend mit Bezug zu der Figur 1 und den Detaildarstellungen der Figuren 2 bis 7 beschrieben.

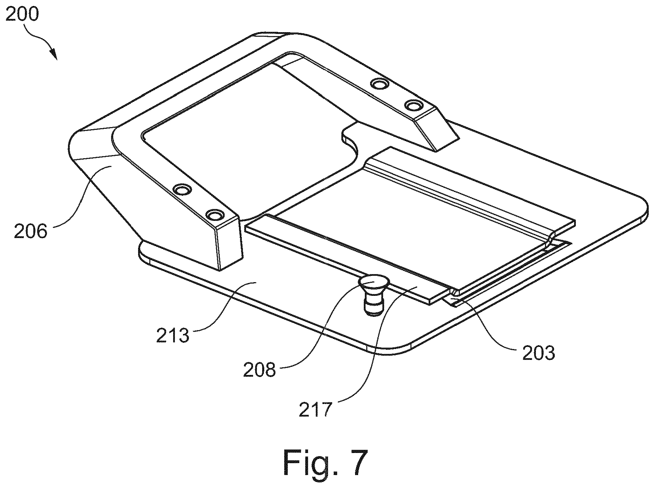

Die Koppeleinheiten 201, 202 umfassen jeweils ein Koppelschwert 203 und eine Koppelplatte 204. Dabei sind die Koppelschwerter 203 hier an dem Koppelelement 205 befestigt. Die Verbindung von Koppelschwert 203 und Koppelplatte 204 ist in der Figur 2 besonders gut zu erkennen, welche diese Verbindung für das obere Gerät 1 in einer Rückansicht des Systems 300 zeigt. Die Koppelplatten 204 sind hier als Zubehörteile an den ersten Aufstellflächen 12 der Gehäuseeinrichtungen 2 der jeweiligen Atemtherapiegeräte 1 befestigt. Durch Einschieben des Koppelschwerts 203 in die Koppelplatte 204 erfolgt die Befestigung der Geräte 1 an dem Koppelelement 205. Dabei ist vorzugsweise ein werkzeugloses Lösen und Befestigen vorgesehen. Zudem kann eine Sicherung, beispielsweise über eine Verrastung, vorgesehen sein. Hier ist beispielsweise ein Sicherungselement 208 vorgesehen, welches in der Figur 7 besonders gut zu erkennen ist.

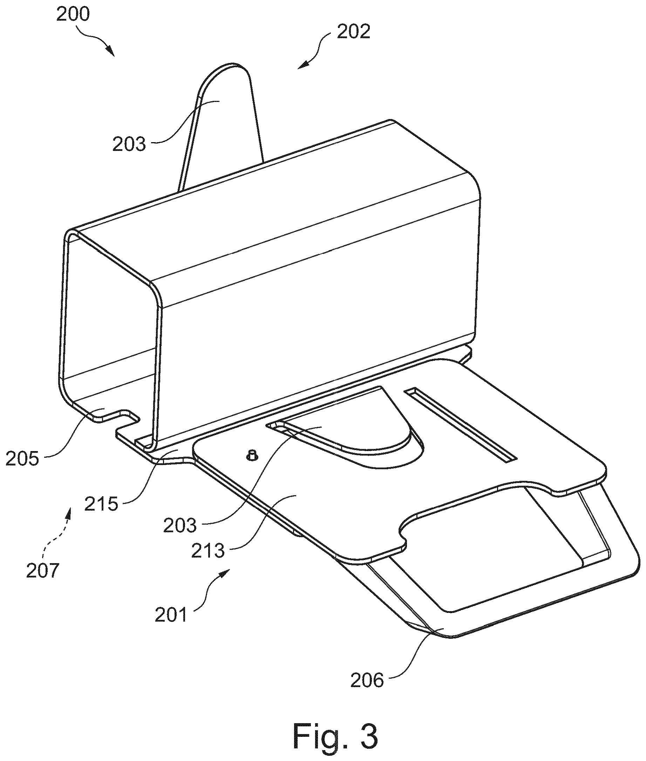

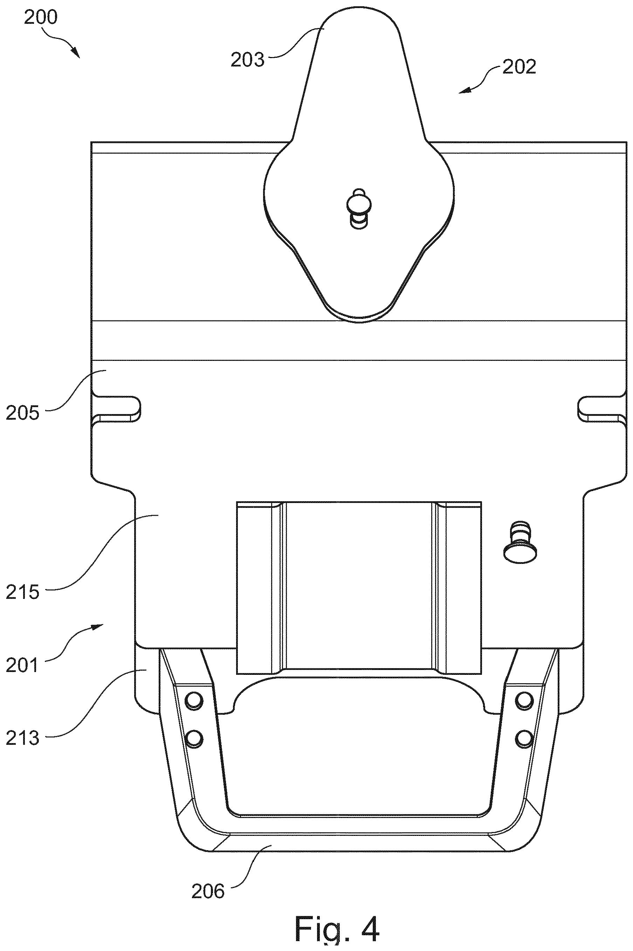

Das Koppelschwert 203 für das untere bzw. liegende Gerät 1 ist hier an einem sich von einer Längsseite des Koppelelements erstreckenden Fortsatz 215 befestigt. Der Fortsatz 215 ist in den Figuren 3 und 4 besonders gut zu erkennen, in denen die Koppeleinrichtung 300 ohne Atemtherapiegeräte 1 gezeigt ist.

Das Koppelschwert 203 für das obere Gerät 1 ist, wie in der Figur 2 besonders gut zu erkennen, an dem Koppelelement 205 montiert. Das Koppelschwert für das untere Gerät 1 ist, wie in der Figur 3 besonders gut zu erkennen, an einer Montageplatte 213 angeordnet. Über die Montageplatte 213 wird dann das Koppelschwert 203 an dem Fortsatz 215 montiert.

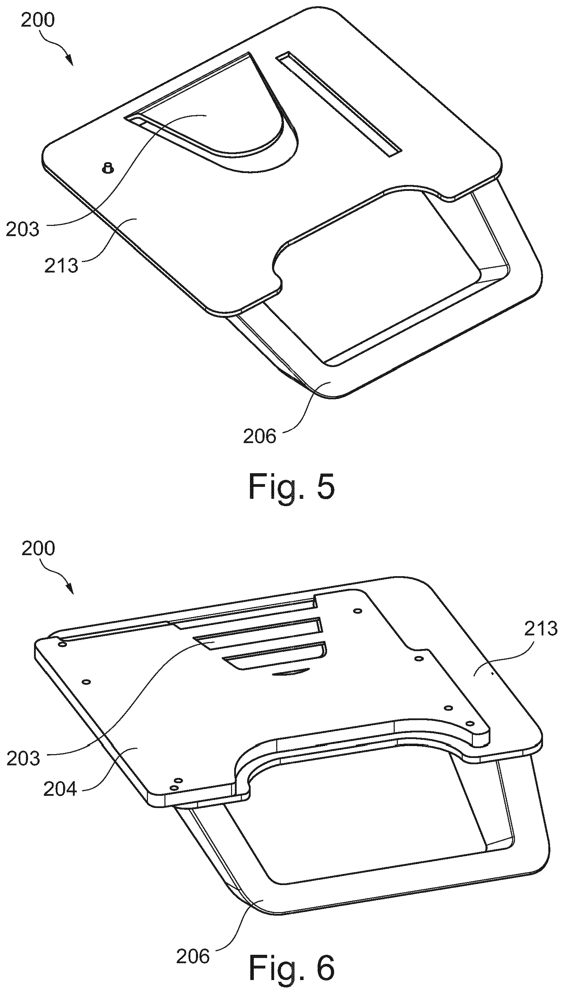

In den Figuren 5 bis 7 ist die Montageplatte 213 mit dem Koppelschwert 203 und dem Zusatzgriff 206 der Koppeleinrichtung 200 aus verschiedenen Ansichten dargestellt. Dabei ist die Montageplatte 213 der Figur 6 in der Figur 7 mit einer Koppelplatte 204 ausgestattet, sodass die Verbindung von Koppelschwert 203 Koppelplatte 204 besonders gut zu erkennen ist. Die Koppelplatte 204 kann Teil der Koppeleinrichtung 200 sein oder auch ein Zubehörteil oder festes Teil der Gehäuseeinrichtung 2 des Atemtherapiegeräts 1 sein. Beispielsweise ist die Koppelplatte 204 an der Gehäuseeinrichtung 2 einschraubbar. Alternativ kann die Koppelplatte 204 auch als eine entsprechende Aussparung in der Gehäuseeinrichtung 2 ausgebildet sein, in welche das Koppelschwert 203 einschiebbar ist. Entsprechende Ausführungen sind auch für die in der Figur 2 gezeigte Koppelplatte 204 des oberen Atemtherapiegeräts 1 vorgesehen.

In der Figur 7 ist die Unterseite der Montageplatte 213 gezeigt, sodass hier eine Verbindungseinrichtung 217 besonders gut zu erkennen ist. Die Verbindungseinrichtung 217 kann zur Verbindung von Koppelplatte 204 und Koppelschwert 203 vorgesehen sein oder auch zur Verbindung mit einem Fahrgestell 207 dienen.

Die Figur 8 zeigt ein einzelnes Atemtherapiegerät 1 in einer liegenden Aufstellposition 3. Das Gerät 1 ist hier noch mit einer Koppelplatte und einem Zusatzgriff 206 ausgestattet, welche jedoch zu der zuvor beschriebenen Koppeleinrichtung 200 gehören und nicht Teil des hier gezeigten Atemtherapiegeräts 1 sind. Das hier gezeigte Gerät 1 kann durch Kippen um die Kippkante 6 einfach und komfortabel in die stehende Aufstellposition 4 überführt werden.

Die Koppeleinrichtung 200 kann in einer Ausführungsform eine Montageplatte 213 für ein Atemtherapiegerät 1 aufweisen, wobei die Koppeleinrichtung 200 mit der Montageplatte 213 auf einem Fahrgestell 207 montiert sind. Die Montageplatte 213 weist zumindest eine Verbindungseinrichtung 217 auf, die als (paariges) Schienensystem ausgeführt sein kann, wobei die Verbindungseinrichtung 217 eingerichtet und ausgebildet ist mit einer korrespondierenden Aufnahme an dem Atemtherapiegerät 1 im wesentlichen kraft- oder formschlüssig zu interagieren. Das Atemtherapiegerät weist dazu im Bereich seiner Aufstellfläche solche Aufnahmen auf, die kraft- oder formschlüssig mit dem Schienensystem interagieren. Das Atemtherapiegerät kann mit seiner Aufstellfläche auf die Montageplatte gestellt werden und auf der Montageplatte in eine Aufstellposition geschoben werden, wobei sich das Atemtherapiegerät zumindest abschnittsweise entlang der Schienen bewegt bis die Schienen mit den Aufnahmen (kraft- oder formschlüssig) interagieren. Ein Sicherungselement 208 im Bereich der Montageplatte dient dazu, dass Atemtherapiegerät 1 auf der Montageplatte in der Aufstellposition zu sichern. Die Montageplatte ist bevorzugt horizontal an einem Fahrgestell angeordnet, wobei sich das Fahrgestell relativ zu der Montageplatte in vertikaler Richtung erstrecken kann. Die Montageplatte weist als linke und rechte Begrenzung die Schienen, in Form einer Erhöhung auf und als hintere Begrenzung eine weitere Schiene oder Kante oder einen Abschnitt des Fahrgestells. Der vordere Bereich der Montageplatte weist keine Begrenzung auf und dient dazu, dass Atemtherapiegerät 1 hier abzustellen und auf die Montageplatte zu schieben.

Die Verbindungseinrichtung 217 und die korrespondierende Aufnahme an dem Atemtherapiegerät 1 sind bevorzugt so eingerichtet und ausgebildet, dass sich beide aneinander ausrichten oder aneinander entlanggleiten, wenn das Atemtherapiegerät mit seiner Aufstellfläche auf die Montageplatte gestellt ist und auf der Montageplatte in eine Aufstellposition geschoben wird. Dazu sind die Schienen bevorzugt trichterförmig auf der horizontal angeordneten Montageplatte angeordnet, wobei die Öffnung des Trichters vorne ist und wobei sich der Trichter nach hinten verengt. Das Atemtherapiegerät kann dann auf der Montageplatte mittels eines Sicherungselementes 208 arretiert werden.

### Bezugszeichenliste:

| | | | |
|---|---|---|---|
| 1 | Atemtherapiegerät | 101 | Akkueinrichtung |
| 2 | Gehäuseeinrichtung | 102 | Lüftereinrichtung |
| 3 | Aufstellposition, liegend | 103 | Schnittstelle |
| 4 | Aufstellposition, stehend | 104 | Kabelanschluss |
| 5 | Griffeinrichtung | 105 | Steuereinrichtung |
| 6 | Kippkante | 200 | Koppeleinrichtung |
| 7 | Anzeigeeinrichtung | 201 | Koppeleinheit |
| 10 | Hustengerät | 202 | Koppeleinheit |
| 12 | Aufstellfläche | 203 | Koppelschwert |
| 17 | Bedieneinrichtung | 204 | Koppelplatte |
| 20 | Beatmungsgerät | 205 | Koppelelement |
| 22 | Aufstellfläche | 206 | Zusatzgriff |
| 32 | Vorderseite | 207 | Fahrgestell |
| 42 | Oberseite | 208 | Sicherungselement |
| 52 | Unterseite | 213 | Montageplatte |
| 62 | Rückseite | 215 | Fortsatz |
| 72 | Seite | 217 | Verbindungseinrichtung |
| 82 | Seite | 300 | System |

## Patentansprüche

1. Atemtherapiegerät (1) zum Erzeugen einer Atemluftströmung für die Durchführung einer Atemtherapie, umfassend wenigstens eine Gehäuseeinrichtung (2) zur Einhausung von Gerätekomponenten, **dadurch gekennzeichnet, dass** die Gehäuseeinrichtung (2) wenigstens zwei Aufstellflächen (12, 22) umfasst, sodass das Atemtherapiegerät (1) in wenigstens zwei Aufstellpositionen (3, 4) bestimmungsgemäß aufstellbar und betreibbar ist, **dadurch gekennzeichnet, dass** ein Geräteschwerpunkt des Atemtherapiegerätes (1) wenigstens in derjenigen der wenigstens zwei Aufstellpositionen (3, 4) in einer unteren Gerätehälfte angeordnet ist, in welcher das Atemtherapiegerät eine größere Aufstellhöhe aufweist, und dass der Geräteschwerpunkt wenigstens durch die Anordnung wenigstens einer Akkueinrichtung (101) zur Energieversorgung des Atemtherapiegeräts (1) und/oder wenigstens einer Lüftereinrichtung (102) zur Erzeugung einer Atemgasströmung bereitgestellt wird.

2. Atemtherapiegerät (1) nach dem vorhergehenden Anspruch, wobei die Gehäuseeinrichtung (2) wenigstens eine erste Aufstellfläche (12) für wenigstens eine erste Aufstellposition (3) und wenigstens eine zweite Aufstellfläche (22) für wenigstens eine zweite Aufstellposition (4) umfasst.

3. Atemtherapiegerät (1) nach dem vorhergehenden Anspruch, wobei die erste Aufstellfläche (12) für eine liegende Aufstellposition (3) und wobei die zweite Aufstellfläche (22) für eine stehende Aufstellposition (4) geeignet und ausgebildet ist, wobei die Aufstellflächen (12, 22) durch jeweils eine Seitenfläche (32, 42, 52, 62, 72, 82) der Gehäuseeinrichtung (2) bereitgestellt werden.

4. Atemtherapiegerät (1) nach dem vorhergehenden Anspruch, wobei in der liegenden Aufstellposition (3) eine Aufstellhöhe des Atemtherapiegeräts (1) geringer als eine Länge und/oder Breite der ersten Aufstellfläche ist und/oder wobei in der stehenden Aufstellposition eine Aufstellhöhe des Atemtherapiegeräts (1) größer als eine Länge und/oder Breite der zweiten Aufstellfläche ist.

5. Atemtherapiegerät (1) nach einem der vorhergehenden Ansprüche, wobei die Aufstellflächen (12, 22) in einem Winkel von wenigstens 65° und maximal 115° und vorzugsweise rechtwinkelig zueinander angeordnet sind.

6. Atemtherapiegerät (1) nach einem der vorhergehenden Ansprüche, umfassend wenigstens eine Schnittstelle (103) zum Koppeln von Schläuchen für eine Atemgasströmung und/oder wenigstens einen Kabelanschluss (104), wobei die Schnittstelle (103) und/oder der Kabelanschluss (104) wenigstens in derjenigen der wenigstens zwei Aufstellpositionen (3, 4), in welcher das Atemtherapiegerät (1) eine größere Aufstellhöhe aufweist, in einer unteren Gerätehälfte angeordnet ist, vorzugsweise wobei die Schnittstelle (103) und/oder der Kabelanschluss (104) in allen bestimmungsgemäßen Aufstellpositionen (3, 4) seitlich an der Gehäuseeinrichtung (2) angeordnet sind.

7. Atemtherapiegerät (1) nach einem der vorhergehenden Ansprüche, wobei wenigstens eine Griffeinrichtung (5) derart angeordnet ist, dass die Griffeinrichtung (5) in einer liegenden Aufstellposition (3) an einer Vorderseite (32) und in einer stehenden Aufstellposition (4) an einer Oberseite (42) der Gehäuseeinrichtung (2) befindlich ist, sodass die Griffeinrichtung (5) in beiden Aufstellpositionen (3, 4) von einer vor dem Atemtherapiegerät (1) stehenden Benutzerperson erreichbar ist.

8. Atemtherapiegerät (1) nach einem der vorhergehenden Ansprüche, wobei die Gehäuseeinrichtung (2) wenigstens eine als Kippkante (6) ausgebildete Gehäusekante aufweist und wobei die Kippkante (6) nachgiebig und/oder verstärkt und/oder abgerundet ausgebildet ist.

9. Atemtherapiegerät (1) nach einem der vorhergehenden Ansprüche, dazu geeignet und ausgebildet, nur durch Aufstellen, vorzugsweise durch Kippen der Gehäuseeinrichtung (2), und ohne weitere Maßnahmen zwischen den Aufstellpositionen (3, 4) bestimmungsgemäß überführt zu werden.

10. Atemtherapiegerät (1) nach einem der vorhergehenden Ansprüche, dazu geeignet und ausgebildet, mittels wenigstens einer Steuereinrichtung (105) die eingenommene Aufstellposition (3, 4) selbstständig zu erkennen und vorzugsweise mittels der Steuereinrichtung (105) in Abhängigkeit der Aufstellposition (3, 4) wenigstens eine Geräteeinstellung zu verändern.

11. Atemtherapiegerät (1) nach dem vorhergehenden Anspruch, wobei in Abhängigkeit der Aufstellposition (3, 4) wenigstens ein Darstellungsmodus einer Anzeigeeinrichtung (7) und/oder wenigstens eine Funktionsbelegung einer Bedieneinrichtung (17) eingestellt wird.

12. Atemtherapiegerät (1) nach einem der vorhergehenden Ansprüche, wobei wenigstens eine Anzeigeeinrichtung (7) und/oder Bedieneinrichtung (17) an wenigstens einer Seitenfläche (32, 42, 52, 62, 72, 82) der Gehäuseeinrichtung (2) angeordnet ist, welche flächenmäßig die größte Seitenfläche (32, 42, 52, 62, 72, 82) der Gehäuseeinrichtung (2) bildet und/oder wobei die Anzeigeeinrichtung (7) und/oder Bedieneinrichtung (17) an wenigstens einer Seitenfläche (32, 42, 52, 62, 72, 82) angeordnet ist, welche eine größere Fläche als die jeweiligen Flächen der Aufstellflächen (12, 22) aufweist.

13. Atemtherapiegerät (1) nach dem vorhergehenden Anspruch, wobei die Anzeigeeinrichtung (7) wenigstens 70 % und insbesondere wenigstens 95% der Seitenfläche (32, 42, 52, 62, 72, 82) der Gehäuseeinrichtung (2) einnimmt.

14. Atemtherapiegerät (1) nach einem der beiden vorhergehenden Ansprüche, wobei die Anzeigeeinrichtung (7) wenigstens eine berührungsempfindliche Oberfläche als Bedieneinrichtung (17) umfasst und wobei die Bedieneinrichtung (17) wenigstens in einem oberen Viertel und vorzugsweise wenigstens in einem oberen Achtel mit geringeren Kräften betätigbar ist, als die zum Kippen der Gehäuseeinrichtung notwendigen Kräfte.

15. Atemtherapiegerät (1) nach einem der drei vorhergehenden Ansprüche, wobei die Anzeigeeinrichtung (7) gegenüber der ersten Aufstellfläche (12) zwischen 0° und 55° geneigt ist und/oder wobei die Anzeigeeinrichtung (7) gegenüber der zweiten Aufstellfläche (22) zwischen 40° und 90° geneigt ist.

16. Atemtherapiegerät (1) nach einem der vier vorhergehenden Ansprüche, wobei ein Betrachtungswinkel der Anzeigeeinrichtung (7) in einer liegenden Aufstellposition (3) für eine stehende Person angepasst ist und/oder wobei ein Betrachtungswinkel der Anzeigeeinrichtung (7) in einer stehenden Aufstellposition (4) für eine liegende bzw. sitzende Person angepasst ist.

17. Atemtherapiegerät (1) nach einem der vorhergehenden Ansprüche, wobei auf der Seitenfläche, in der die Griffeinrichtung angeordnet ist, mindestens ein weiteres Bedienelement angeordnet ist.

18. Atemtherapiegerät (1) nach einem der vorhergehenden Ansprüche, dazu geeignet und ausgebildet, eine betriebsgemäß erforderliche Atemluftströmung unabhängig von der Aufstellposition (3, 4) bereitzustellen.

19. Atemtherapiegerät (1) nach einem der vorhergehenden Ansprüche, wobei alle bestimmungsgemäß vorgesehenen Öffnungen der Gehäuseeinrichtung (2) in allen bestimmungsgemäßen Aufstellpositionen (3, 4) wenigstens gegen feste Fremdkörper mit Durchmesser ≥ 12,5 mm und gegen den Zugang mit einem Finger geschützt sind und einen Schutz gegen fallendes Tropfwasser, wenn die Gehäuseeinrichtung (2) bis zu 15° geneigt ist, aufweisen.

20. Atemtherapiegerät (1) nach einem der vorhergehenden Ansprüche, ausgebildet als tragbares Atemtherapiegerät (1) mit einem maximalen Gewicht von 10 kg und vorzugsweise 6 kg.

21. Atemtherapiegerät (1) nach einem der vorhergehenden Ansprüche, wobei die Gehäuseeinrichtung (2) dazu geeignet und ausgebildet ist, in wenigstens einer der Aufstellpositionen (3, 4) mit einer Gehäuseeinrichtung (2) eines weiteren erfindungsgemäßen Atemtherapiegeräts (1) in einer sich gegenseitig abstützenden Anordnung aufgestellt und betrieben zu werden.

22. Atemtherapiegerät (1) nach einem der vorhergehenden Ansprüche, ausgebildet als Hustengerät (10) und/oder Beatmungsgerät (20).

23. System umfassend wenigstens zwei Atemtherapiegeräte (1) nach einem der vorhergehenden Ansprüche und eine Koppeleinrichtung (200) zur Kopplung von den wenigstens zwei Atemtherapiegeräten (1), wobei die Koppeleinrichtung ferner umfasst wenigstens zwei über ein Koppelelement (205) miteinander verbindbare Koppeleinheiten (201, 202), wobei an wenigstens einer Koppeleinheit (201) das eine Atemtherapiegerät (1) mittels dessen Gehäuseeinrichtung (2) liegend aufnehmbar ist und wobei an wenigstens einer anderen Koppeleinheit (202) das andere Atemtherapiegerät (1) mittels dessen Gehäuseeinrichtung (2) stehend aufnehmbar ist, sodass die Atemtherapiegeräte (1) in unterschiedlichen Aufstellpositionen (3, 4) gleichzeitig an der Koppeleinrichtung (200) aufnehmbar und daran bestimmungsgemäß betreibbar sind.

24. System aufweisend wenigstens zwei Atemtherapiegeräte (1) nach einem der vorhergehenden Ansprüche und zumindest eine Koppeleinrichtung (200) zur Kopplung von den wenigstens zwei Atemtherapiegeräten (1) wobei das eine Atemtherapiegerät (1) als Beatmungsgerät (20) und das andere als Hustengerät (10) ausgebildet ist.

## Claims

1. A respiratory therapy device (1) for generating a breathing gas flow for performing respiratory therapy, comprising at least one housing apparatus (2) for enclosing device components, **characterized in that** the housing apparatus (2) comprises at least two placement surfaces (12, 22), such that the respiratory therapy device (1) can be placed and operated as intended in at least two placement positions (3, 4), **characterized in that** a device center of gravity of the respiratory therapy device (1) is disposed in a lower half of the device, at least in the one of the at least two placement positions (3, 4) in which the respiratory therapy device has a greater placement height, and **in that** the device center of gravity is provided at least by the disposition of at least one battery apparatus (101) for supplying energy to the respiratory therapy device (1) and/or at least one fan apparatus (102) for generating a breathing gas flow.

2. The respiratory therapy device (1) according to the preceding claim, wherein the housing apparatus (2) comprises at least one first placement surface (12) for at least one first placement position (3) and at least one second placement surface (22) for at least one second placement position (4).

3. The respiratory therapy device (1) according to the preceding claim, wherein the first placement surface (12) is suitable and designed for a horizontal placement position (3) and wherein the second placement surface (22) is suitable and designed for a vertical placement position (4), wherein the placement surfaces (12, 22) are respectively provided by a side surface (32, 42, 52, 62, 72, 82) of the housing apparatus (2).

4. The respiratory therapy device (1) according to the preceding claim, wherein, in the horizontal placement position (3), an placement height of the respiratory therapy device (1) is less than a length and/or a width of the first placement surface and/or wherein, in the vertical placement position, an placement height of the respiratory therapy device (1) is greater than a length and/or a width of the second placement surface.

5. The respiratory therapy device (1) according to one of the preceding claims, wherein the placement surfaces (12, 22) are disposed at an angle of at least 65° and at most 115°, and preferably at right angles, to one another.

6. The respiratory therapy device (1) according to one of the preceding claims, comprising at least one interface (103) for coupling hoses for a breathing gas flow and/or at least one cable connection (104), wherein the interface (103) and/or the cable connection (104) is disposed in a lower half of the device, at least in the one of the at least two placement positions (3, 4) in which the respiratory therapy device (1) has a greater placement height, preferably wherein the interface (103) and/or the cable connection (104) are disposed laterally on the housing apparatus (2) in all intended placement positions (3, 4).

7. The respiratory therapy device (1) according to one of the preceding claims, wherein at least one gripping apparatus (5) is disposed such that the gripping apparatus (5) is located, in a horizontal placement position (3), on a front side (32) and, in a vertical placement position (4), on a top side (42) of the housing apparatus (2), such that the gripping apparatus (5) is accessible in both placement positions (3, 4) by a user standing in front of the respiratory therapy device (1).

8. The respiratory therapy device (1) according to one of the preceding claims, wherein the housing apparatus (2) has at least one housing edge designed as a tilt edge (6), and wherein the tilt edge (6) is designed to be yielding and/or reinforced and/or rounded.

9. The respiratory therapy device (1) according to one of the preceding claims, which is suitable and designed for being transferred as intended between the placement positions (3, 4) solely by being placed, preferably by tilting of the housing apparatus (2), and without further measures.

10. The respiratory therapy device (1) according to one of the preceding claims, which is suitable and designed, by means of at least one control apparatus (105), for automatically detecting the placement position (3, 4) assumed and preferably, by means of the control apparatus (105), for changing at least one device setting depending on the placement position (3, 4).

11. The respiratory therapy device (1) according to the preceding claim, wherein at least one display mode of a display apparatus (7) and/or at least one function assignment of an operating apparatus (17) is set depending on the placement position (3, 4).

12. The respiratory therapy device (1) according to one of the preceding claims, wherein at least one display apparatus (7) and/or operating apparatus (17) is disposed on at least one side surface (32, 42, 52, 62, 72, 82) of the housing apparatus (2) which, in terms of area, forms the largest side surface (32, 42, 52, 62, 72, 82) of the housing apparatus (2) and/or wherein the display apparatus (7) and/or operating apparatus (17) is disposed on at least one side surface (32, 42, 52, 62, 72, 82) which has a larger area than the respective areas of the placement surfaces (12, 22).

13. The respiratory therapy device (1) according to the preceding claim, wherein the display apparatus (7) occupies at least 70% and, in particular, at least 95% of the side surface (32, 42, 52, 62, 72, 82) of the housing apparatus (2).

14. The respiratory therapy device (1) according to one of the two preceding claims, wherein the display apparatus (7) comprises at least one touch-sensitive surface as an operating apparatus (17), and wherein the operating apparatus (17) can be actuated at least in an upper quarter, and preferably at least in an upper eighth, using forces that are lower than the forces necessary for tilting the housing apparatus.

15. The respiratory therapy device (1) according to one of the three preceding claims, wherein the display apparatus (7) is inclined by between 0° and 55° with respect to the first placement surface (12) and/or wherein the display apparatus (7) is inclined by between 40° and 90° with respect to the second placement surface (22).

16. The respiratory therapy device (1) according to one of the four preceding claims, wherein a viewing angle of the display apparatus (7) in a horizontal placement position (3) is adapted for a standing person and/or wherein a viewing angle of the display apparatus (7) in a vertical placement position (4) is adapted for a recumbent or else seated person.

17. The respiratory therapy device (1) according to one of the preceding claims, wherein at least one further operating element is disposed on the side surface in which the gripping apparatus is disposed.

18. The respiratory therapy device (1) according to one of the preceding claims, which is suitable and designed for providing a breathing air flow required for operation regardless of the placement position (3, 4).

19. The respiratory therapy device (1) according to one of the preceding claims, wherein all openings of the housing apparatus (2) provided for the intended use, in all intended placement positions (3, 4), are at least protected against solid foreign objects having a diameter ≥ 12.5 mm and against access with a finger, and have protection against falling drops of water when the housing apparatus (2) is inclined by up to 15°.

20. The respiratory therapy device (1) according to one of the preceding claims, which is designed as a portable respiratory therapy device (1) having a maximum weight of 10 kg and preferably 6 kg.

21. The respiratory therapy device (1) according to one of the preceding claims, wherein the housing apparatus (2) is suitable and designed for being placed and operated, in at least one of the placement positions (3, 4), with a housing apparatus (2) of a further respiratory therapy device (1) according to the invention in a mutually supporting disposition.

22. The respiratory therapy device (1) according to one of the preceding claims, designed as a cough-assist device (10) and/or a ventilator (20).

23. A system comprising at least two respiratory therapy devices (1) according to one of the preceding claims and a coupling apparatus (200) for coupling the at least two respiratory therapy devices (1), wherein the coupling apparatus further comprises at least two coupling units (201, 202) that can be interconnected via a coupling element (205), wherein one respiratory therapy device (1) can be horizontally received by means of the housing apparatus (2) thereof on at least one coupling unit (201) and wherein the other respiratory therapy device (1) can be vertically received by means of the housing apparatus (2) thereof on at least one other coupling unit (202), such that the respiratory therapy devices (1) can be received on the coupling apparatus (200) in different placement positions (3, 4) at the same time and can be operated thereon as intended.

24. A system having at least two respiratory therapy devices (1) according to one of the preceding claims and at least one coupling apparatus (200) for coupling the at least two respiratory therapy devices (1), wherein one respiratory therapy device (1) is designed as a ventilator (20) and the other is designed as a cough-assist device (10).

## Revendications

1. Appareil de thérapie respiratoire (1) destiné à générer un flux d'air respiratoire pour la mise en œuvre d'une thérapie respiratoire, comprenant au moins un ensemble de boîtier (2) destiné à loger des composants du dispositif, **caractérisé en ce que** l'ensemble de boîtier (2) comprend au moins deux surfaces d'installation (12, 22), de sorte que l'appareil de thérapie respiratoire (1) peut être installé et exploité de manière conforme dans au moins deux positions d'installation (3, 4), et **en ce qu'**un centre de gravité du dispositif (1) est disposé dans une moitié inférieure du dispositif au moins dans celle des au moins deux positions d'installation (3, 4) dans laquelle l'appareil de thérapie respiratoire présente une hauteur d'installation plus grande, et **en ce que** le centre de gravité est assuré au moins par la disposition d'au moins une unité d'accumulateur (101) destinée à l'alimentation en énergie de l'appareil de thérapie respiratoire (1) et/ou d'au moins une unité de ventilateur (102) destinée à générer un flux de gaz respiratoire.

2. Appareil de thérapie respiratoire (1) selon la revendication précédente, dans lequel l'ensemble de boîtier (2) comprend au moins une première surface d'installation (12) pour au moins une première position d'installation (3) et au moins une deuxième surface d'installation (22) pour au moins une deuxième position d'installation (4).

3. Appareil de thérapie respiratoire (1) selon la revendication précédente, dans lequel la première surface d'installation (12) est adaptée et conçue pour une position d'installation horizontale (3) et la seconde surface d'installation (22) pour une position d'installation verticale (4), dans lequel les surfaces d'installation (12, 22) sont formées respectivement par une surface latérale (32, 42, 52, 62, 72, 82) de l'ensemble de boîtier (2).

4. Appareil de thérapie respiratoire (1) selon la revendication précédente, dans lequel, dans la position d'installation horizontale (3), une hauteur d'installation de l'appareil de thérapie respiratoire (1) est inférieure à une longueur et/ou à une largeur de la première surface d'installation et/ou dans laquelle, dans la position d'installation verticale, une hauteur d'installation de l'appareil de thérapie respiratoire (1) est supérieure à une longueur et/ou à une largeur de la deuxième surface d'installation.

5. Appareil de thérapie respiratoire (1) selon l'une des revendications précédentes, dans lequel les surfaces d'installation (12, 22) sont disposées l'une par rapport à l'autre selon un angle d'au moins 65° et de 115° maximum, et de préférence à angle droit.

6. Appareil de thérapie respiratoire (1) selon l'une des revendications précédentes, comprenant au moins une interface (103) destinée au couplage de tuyaux pour un flux de gaz respiratoire et/ou au moins un raccord de câble (104), dans lequel l'interface (103) et/ou le raccord de câble (104) sont disposés dans une moitié inférieure de l'appareil au moins dans celle des au moins deux positions d'installation (3, 4) dans laquelle l'appareil de thérapie respiratoire (1) présente une plus grande hauteur d'installation, dans lequel l'interface (103) et/ou le raccord de câble (104) sont disposés de préférence latéralement sur l'ensemble de boîtier (2) dans toutes les positions d'installation (3, 4) prévues.

7. Appareil de thérapie respiratoire (1) selon l'une des revendications précédentes, dans lequel au moins un dispositif de préhension (5) est disposé de telle sorte que le dispositif de préhension (5) se trouve sur une face avant (32) de l'ensemble de boîtier (2) dans une position d'installation horizontale (3), et sur une face supérieure (42) de celui-ci dans une position d'installation verticale (4), de sorte que dans les deux positions d'installation (3, 4), le dispositif de préhension (5) est accessible par un utilisateur se tenant devant l'appareil de thérapie respiratoire (1).

8. Appareil de thérapie respiratoire (1) selon l'une des revendications précédentes, dans lequel l'ensemble de boîtier (2) présente au moins une arête de boîtier conçue comme une arête de basculement (6), et dans lequel l'arête de basculement (6) est conçue de manière souple et/ou renforcée et/ou arrondie.

9. Appareil de thérapie respiratoire (1) selon l'une des revendications précédentes, adapté et conçu pour être transféré de manière conforme entre les positions d'installation (3, 4) uniquement par installation, de préférence par basculement de l'ensemble de boîtier (2), et sans autre intervention.

10. Appareil de thérapie respiratoire (1) selon l'une des revendications précédentes, adapté et conçu pour reconnaître automatiquement la position d'installation (3, 4) adoptée au moyen d'au moins un dispositif de commande (105), et, de préférence, pour modifier au moins un réglage de l'appareil en fonction de la position d'installation (3, 4) au moyen du dispositif de commande (105).

11. Appareil de thérapie respiratoire (1) selon la revendication précédente, dans lequel au moins un mode d'affichage d'un dispositif d'affichage (7) et/ou au moins une affectation de fonction d'un dispositif de fonctionnement (17) sont réglés en fonction de la position d'installation (3, 4).

12. Appareil de thérapie respiratoire (1) selon l'une des revendications précédentes, dans lequel au moins un dispositif d'affichage (7) et/ou un dispositif de fonctionnement (17) sont disposés sur au moins une surface latérale (32, 42, 52, 62, 72, 82) de l'ensemble de boîtier (2), laquelle constitue, en termes de surface, la plus grande des surfaces latérales (32, 42, 52, 62, 72, 82) de l'ensemble de boîtier (2) et/ou dans lequel le dispositif d'affichage (7) et/ou le dispositif de fonctionnement (17) sont disposés sur au moins une surface latérale (32, 42, 52, 62, 72, 82) présentant une surface plus grande que les surfaces d'installation (12, 22) respectives.

13. Appareil de thérapie respiratoire (1) selon la revendication précédente, dans lequel le dispositif d'affichage (7) occupe au moins 70 % et en particulier au moins 95 % de la surface latérale (32, 42, 52, 62, 72, 82) de l'ensemble de boîtier (2).

14. Appareil de thérapie respiratoire (1) selon l'une des deux revendications précédentes, dans lequel le dispositif d'affichage (7) comprend au moins une surface tactile en tant que dispositif de fonctionnement (17) et dans lequel le dispositif de fonctionnement (17) peut être actionné, au moins dans un quart supérieur et de préférence au moins dans un huitième supérieur, avec des forces inférieures à celles nécessaires pour faire basculer l'ensemble de boîtier.

15. Appareil de thérapie respiratoire (1) selon l'une des trois revendications précédentes, dans lequel le dispositif d'affichage (7) est incliné entre 0° et 55° par rapport à la première surface d'installation (12) et/ou dans lequel le dispositif d'affichage (7) est incliné entre 40° et 90° par rapport à la seconde surface d'installation (22).

16. Appareil de thérapie respiratoire (1) selon l'une des quatre revendications précédentes, dans lequel un angle de vision du dispositif d'affichage (7) dans une position d'installation horizontale (3) est adapté pour une personne se tenant debout et/ou dans lequel un angle de vision du dispositif d'affichage (7) dans une position d'installation verticale (4) est adapté pour une personne allongée ou assise.

17. Appareil de thérapie respiratoire (1) selon l'une des revendications précédentes, dans lequel au moins un élément de fonctionnement supplémentaire est disposé sur la surface latérale sur laquelle est disposé le dispositif de préhension.

18. Appareil de thérapie respiratoire (1) selon l'une des revendications précédentes, adapté et conçu pour fournir un flux d'air respiratoire nécessaire au fonctionnement indépendamment de la position d'installation (3, 4).

19. Appareil de thérapie respiratoire (1) selon l'une quelconque des revendications précédentes, dans lequel, dans toutes les positions d'installation (3, 4) prévues, toutes les ouvertures prévues conformément à l'utilisation de l'ensemble de boîtier (2) sont protégées au moins contre des corps étrangers solides d'un diamètre ≥ 12,5 mm et contre l'accès avec un doigt et présentent une protection contre l'eau tombant en gouttes, lorsque l'ensemble de boîtier (2) est incliné jusqu'à 15°.

20. Appareil de thérapie respiratoire (1) selon l'une quelconque des revendications précédentes, conçu sous la forme d'un appareil de thérapie respiratoire portable (1) ayant un poids maximal de 10 kg et de préférence de 6 kg maximum.

21. Appareil de thérapie respiratoire (1) selon l'une des revendications précédentes, dans lequel, dans au moins l'une des positions d'installation (3, 4), l'ensemble de boîtier (2) est adapté et conçu pour être installé et exploité avec un ensemble de boîtier (2) d'un autre appareil de thérapie respiratoire (1) conforme à l'invention, dans un agencement en appui mutuel.

22. Appareil de thérapie respiratoire (1) selon l'une des revendications précédentes, conçu sous la forme d'un appareil d'assistance à la toux (10) et/ou d'un appareil de ventilation (20).

23. Système comprenant au moins deux appareils de thérapie respiratoire (1) selon l'une des revendications précédentes et un dispositif de couplage (200) destiné au couplage desdits au moins deux appareils de thérapie respiratoire (1), dans lequel le dispositif de couplage comprend en outre au moins deux unités de couplage (201, 202) aptes à être reliées entre elles au moyen d'un élément de couplage (205), dans lequel au moins une unité de couplage (201) est apte à recevoir l'un des appareils de thérapie respiratoire (1) en position horizontale au moyen de son ensemble de boîtier (2) et dans lequel au moins une autre unité de couplage (202) est apte à recevoir l'autre appareil de thérapie respiratoire (1) en position verticale au moyen de son ensemble de boîtier (2), de telle sorte que les appareils de thérapie respiratoire (1) peuvent être reçus simultanément sur le dispositif de couplage (200) dans des positions d'installation (3, 4) différentes et y être exploités de manière conforme.

24. Système présentant au moins deux appareils de thérapie respiratoire (1) selon l'une des revendications précédentes et au moins un dispositif de couplage (200) destiné à coupler lesdits au moins deux appareils de thérapie respiratoire (1), dans lequel l'un des appareils de thérapie respiratoire (1) est conçu en tant qu'appareil de ventilation (20) et l'autre en tant qu'appareil d'assistance à la toux (10).
